(19) Europäisches Patentamt
European Patent Office
Office européen des brevets

(11) EP 4 385 415 A1

(12) # EUROPEAN PATENT APPLICATION
published in accordance with Art. 153(4) EPC

(43) Date of publication:
**19.06.2024 Bulletin 2024/25**

(21) Application number: **22863871.4**

(22) Date of filing: **09.03.2022**

(51) International Patent Classification (IPC):
***A61B 5/374** (2021.01)*

(52) Cooperative Patent Classification (CPC):
**A61B 5/374**

(86) International application number:
**PCT/JP2022/010204**

(87) International publication number:
**WO 2023/032281 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.08.2021 JP 2021139837**

(71) Applicant: **Sony Group Corporation**
**Tokyo**
**108-0075 (JP)**

(72) Inventors:
- **ISHIKAWA, Takanori**
  **Tokyo 108-0075 (JP)**
- **KITAKAMI, Yukinojo**
  **Tokyo 108-0075 (JP)**

(74) Representative: **D Young & Co LLP**
**3 Noble Street**
**London EC2V 7BQ (GB)**

(54) **INFORMATION PROCESSING DEVICE, INFORMATION PROCESSING METHOD, AND PROGRAM**

(57) The present technology relates to an information processing apparatus, an information processing method, and a program that enable reduction of influences of body movement noise contained in electroencephalography signals. The information processing apparatus includes: a body movement noise identification unit that identifies, on the basis of an electroencephalography signal of a user, body movement noise contained in the electroencephalography signal; and a noise reduction processing control unit that controls, on the basis of an identification result for the body movement noise, noise reduction processing of reducing the body movement noise contained in the electroencephalography signal. The present technology can be applied to, for example, a wearable device.

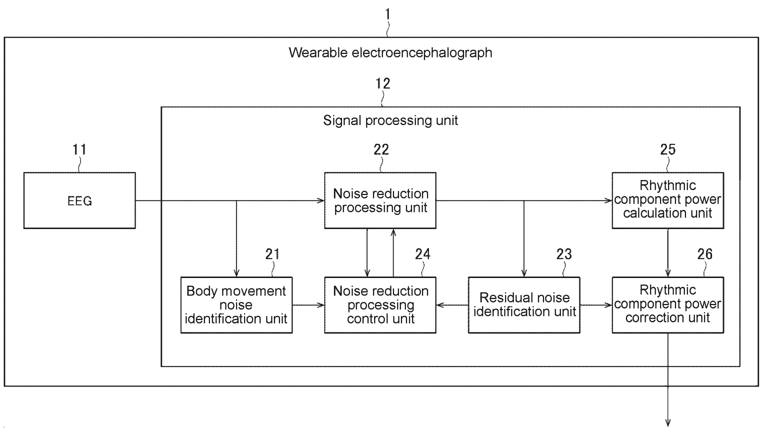


FIG.1



Processed by Luminess, 75001 PARIS (FR)

## Description

### Technical Field

The present technology relates to an information processing apparatus, an information processing method, and a program and more particularly relates to an information processing apparatus, an information processing method, and a program that enable reduction of influences of body movement noise on an electroencephalography signal.

### Background Art

In recent years, various applications such as brain compute interface (BCI) and concentration and relax estimation making use of rhythmic components in electroencephalography signals have been proposed. For example, a system for discriminating subject's sleeping stages by using electroencephalography signals has been proposed (e.g., see Patent Literature 1).
On the other hand, in a case of measuring an electroencephalogram in a daily life, noise (hereinafter, referred to as body movement noise) caused by various body movements such as eye blinks, electromyogram, changes in contact impedance between electrodes and human, electrocardiogram, and sweating is superimposed on the electroencephalography signals. Then, the body movement noise causes application failures (e.g., malfunction, erroneous detection, lowering of accuracy, etc.)
In this context, various reduction methods for the body movement noise have been proposed. For example, Patent Literature 1 has proposed a method of using empirical mode decomposition (EMD) for separating signal components related to brain activity from signal components related to heart activity that are noise components.

### Citation List

### Patent Literature

Patent Literature 1: Japanese UnexaminedPatent Application Publication No. 2019-503746

## Disclosure of Invention

### Technical Problem

However, the frequency range of the body movement noise and the frequency range of the electroencephalogram often overlap, so it is difficult to completely separate the body movement noise. Then, body movement noise (hereinafter, referred to as residual noise) remaining without being separated from the electroencephalography signals becomes a factor for the application failures.
For example, in a case where observed signals are decomposed into a plurality of signal components (singular mode functions) in the EMD, a mode mixing problem in that signal components in the same frequency range are decomposed into the plurality of singular mode functions arises. The mode mixing problem makes it difficult to correctly separate only the body movement noise from the electroencephalography signals, and the residual noise associated with the mode mixing problem becomes a factor for the application failures.
It should be noted that for example also with principal component analysis (PCA), independent component analysis (ICA), singular spectrum analysis (SSA), and the like other than the EMD, it is difficult to completely separate signals derived of the brain activity from the body movement noise.
The present technology has been made in view of such circumstances for enabling reduction of influences of body movement noise on an electroencephalography signal.

### Solution to Problem

An information processing apparatus according to an aspect of the present technology includes: a body movement noise identification unit that identifies, on the basis of an electroencephalography signal of a user, body movement noise contained in the electroencephalography signal; and a noise reduction processing control unit that controls, on the basis of an identification result for the body movement noise, noise reduction processing of reducing the body movement noise contained in the electroencephalography signal.
An information processing method according to an aspect of the present technology includes: identifying, on the basis of an electroencephalography signal of a user, body movement noise contained in the electroencephalography signal; and controlling, on the basis of an identification result for the body movement noise, noise reduction processing of reducing the body movement noise contained in the electroencephalography signal.

A program according to an aspect of the present technology causes a computer to execute processing of: identifying, on the basis of an electroencephalography signal of a user, body movement noise contained in the electroencephalography signal; and controlling, on the basis of an identification result for the body movement noise, noise reduction processing of reducing the body movement noise contained in the electroencephalography signal.

In an aspect of in the present technology, the body movement noise contained in the electroencephalography signal is identified on the basis of the electroencephalography signal of the user; and the noise reduction processing of reducing the body movement noise contained in the electroencephalography signal controlled on the basis of the identification result for the body movement noise.

Brief Description of Drawings

[Fig. 1] A block diagram showing a first embodiment of a wearable electroencephalograph to which the present technology is applied.
[Fig. 2] A block diagram showing a configuration example of a learning apparatus to which the present technology is applied.
[Fig. 3] A block diagram showing a configuration example of a body movement noise identification unit.
[Fig. 4] A block diagram showing a configuration example of a residual noise identification unit.
[Fig. 5] A block diagram showing a first embodiment of a noise reduction processing unit.
[Fig. 6] A flowchart for describing signal processing executed by the wearable electroencephalograph in Fig. 1.
[Fig. 7] A flowchart for describing a first embodiment of noise reduction processing.
[Fig. 8] A block diagram showing a second embodiment of the noise reduction processing unit.
[Fig. 9] A flowchart for describing a second embodiment of the noise reduction processing.
[Fig. 10] A block diagram showing a second embodiment of the wearable electroencephalograph to which the present technology is applied.
[Fig. 11] A flowchart for describing a first embodiment of the signal processing executed by the wearable electroencephalograph in Fig. 10.
[Fig. 12] A flowchart for describing a second embodiment of the signal processing executed by the wearable electroencephalograph in Fig. 10.
[Fig. 13] A block diagram showing a configuration example of a computer.

Mode(s) for Carrying Out the Invention

Hereinafter, modes for carrying out the present technology will be described. The descriptions will be made in the following order.

1. First Embodiment
2. Second Embodiment
3. Third Embodiment
4. Modified Examples
5. Others

<<1. First Embodiment>>

First of all, a first embodiment of the present technology will be described with reference to Figs. 1 to 7.

<Configuration Example of Wearable Electroencephalograph 1>

Fig. 1 is a block diagram showing a configuration example of a wearable electroencephalograph 1 that is a first embodiment of a wearable electroencephalograph to which the present technology is applied.

The wearable electroencephalograph 1 is an apparatus which is attached to a user and measures an electroencephalogram of the user. The wearable electroencephalograph 1 includes an electroencephalograph (EEG) 11 and a signal processing unit 12. The signal processing unit 12 includes a body movement noise identification unit 21, a noise reduction processing unit 22, a residual noise identification unit 23, a noise reduction processing control unit 24, a rhythmic component power calculation unit 25, and a rhythmic component power correction unit 26.

The EEG 11 measures an electroencephalogram of the user with electrodes attached to predetermined sites of the user. The EEG 11 supplies an electroencephalography signal indicating a measurement result to the body movement noise identification unit 21 and the noise reduction processing unit 22.

It should be noted that sites where the EEG 11 measures the electroencephalogram of the user is not particularly limited. For example, electrodes for measuring the EEG 11 are attached to the user's scalp (scalp EEG), inner parts of the ear (in-ear EEG), or peripheral parts of the ear (around-ear EEG) for measuring the electroencephalogram of the user.

The body movement noise identification unit 21 identifies body movement noise on the basis of the electroencephalography signal. For example, the body movement noise identification unit 21 identifies the presence/absence and the type of body movement noise contained in the electroencephalography signal. The body movement noise identification unit 21 supplies body movement noise information indicating an identification result for the body movement noise to the noise reduction processing control unit 24.

Under the control of the noise reduction processing control unit 24, the noise reduction processing unit 22 executes noise reduction processing of reducing the body movement noise contained in the electroencephalography signal. The noise reduction processing unit 22 supplies the electroencephalography signal after the noise reduction processing to the residual noise identification unit 23 and the rhythmic component power calculation unit 25. Moreover, the noise reduction processing unit 22 supplies analysis information indicating an analysis result for the electroencephalography signal executed in the noise reduction processing to the noise reduction processing control unit 24.

On the basis of the electroencephalography signal after the noise reduction processing, the residual noise identification unit 23 identifies residual noise which is body movement noise remaining without being removed from the electroencephalography signal. For example, the body movement noise identification unit 21 identifies the presence/absence and the type of residual noise contained in the electroencephalography signal. The residual noise identification unit 23 supplies residual noise information indicating an identification result for the residual noise to the noise reduction processing control unit 24 and the rhythmic component power correction unit 26.

The noise reduction processing control unit 24 controls the noise reduction processing by the noise reduction processing unit 22 on the basis of at least one of the body movement noise information, the residual noise information, and the analysis information. For example, the noise reduction processing control unit 24 sets a discrimination parameter used for discriminating components of the body movement noise contained in the electroencephalography signal on the basis of the analysis result for the electroencephalography signal and supplies the discrimination parameter to the noise reduction processing unit 22.

The rhythmic component power calculation unit 25 calculates rhythmic component power which is power in a rhythmic component frequency range in the electroencephalography signal. The rhythmic component power calculation unit 25 supplies rhythmic component power information indicating a calculation result for the rhythmic component power to the rhythmic component power correction unit 26.

On the basis of the residual noise information, the rhythmic component power correction unit 26 corrects the rhythmic component power calculated by the rhythmic component power calculation unit 25. Moreover, the rhythmic component power correction unit 26 calculates reliability for the rhythmic component power on the basis of the residual noise information. The rhythmic component power correction unit 26 outputs output information including the corrected rhythmic component power and the reliability.

<Configuration Example of Learning Apparatus 51>

Fig. 2 shows a configuration example of a learning apparatus 51 that executes learning of a body movement noise identification model used by the body movement noise identification unit 21.

The learning apparatus 51 includes a learning data accumulation unit 61, an analysis window 62, a time feature calculation unit 63, a frequency feature calculation unit 64, and a model learning unit 65.

The learning data accumulation unit 61 accumulates learning data to be used in the learning of the body movement noise identification models. The learning data is constituted by a dataset group including input data and training data.

The input data includes an electroencephalography signal indicating a measurement result by the EEG 11 of the wearable electroencephalograph 1, for example.

The training data includes a body movement noise label (expected value) indicating the presence/absence and the type of body movement noise contained in the electroencephalography signal contained in the input data. The body movement noise label is applied by, for example, subjective assessment with respect to the corresponding electroencephalography signal. The body movement noise label is classified as, for example, 0: no noise, 1: eye blink noise, 2: electromyography noise, 3: contact impedance change noise, or 4: others.

The analysis window 62 sequentially acquires input data (electroencephalography signal) included in the learning data accumulated in the learning data accumulation unit 61. The analysis window 62 cuts out the electroencephalography signal while shifting an interval (hereinafter, referred to as an analysis window interval) for cutting out the electroencephalography signal in a time axis direction by using a window function. The analysis window 62 supplies the cut-out electroencephalography signal to the time feature calculation unit 63 and the frequency feature calculation unit 64.

The time feature calculation unit 63 calculates a time feature of the electroencephalography signal supplied from the analysis window 62. Although the type of calculated time feature and the number of calculated time features are not particularly limited, for example, average and variance as basic statistics, kurtosis and skewness as higher-order statistics, entropy as an information amount, or the like are used for the time feature. The time feature calculation unit 63 supplies a time feature of the electroencephalography signal to the model learning unit 65.

The frequency feature calculation unit 64 calculates a frequency feature of the electroencephalography signal supplied from the analysis window 62. Although the type of calculated frequency feature and the number of calculated frequency features are not particularly limited, for example, power spectrum or the like based on short-time Fourier transform is used for the frequency feature. The frequency feature calculation unit 64 supplies the time feature of the electroencephalography signal to the model learning unit 65.

The model learning unit 65 acquires training data (body movement noise label) corresponding to the input data from the learning data accumulation unit 61. On the basis of the time feature and the frequency feature of the input data (electroencephalography signal), the model learning unit 65 learns the body movement noise identification model for estimating the body movement noise label. It should be noted that a machine learning method used for learning the body movement noise identification model is not particularly limited. For example, a machine learning method such as random forest (RF), support vector machine (SVM), or deep neural network (DNN) is used.

As it will be described later, the body movement noise identification model learned by the learning apparatus 51 is used in the body movement noise identification unit 21 of the wearable electroencephalograph 1.

It should be noted that the same body movement noise identification model as that for the body movement noise identification unit 21 or a dedicated learning model may be used as a residual noise identification model used in the residual noise identification unit 23.

In the latter case, the learning of the residual noise identification model is performed by, for example, a learning method similar to that for the body movement noise identification model.

In this case, for example, the electroencephalography signal subjected to the noise reduction processing by the noise reduction processing unit 22 of the wearable electroencephalograph 1 is used as input data for the learning data. Moreover, for example, a residual noise label (expected value) indicating the presence/absence and the type of residual noise contained in the electroencephalography signal after the noise reduction processing included in the input data is used as training data for the learning data. The residual noise label is classified in a similar way to the body movement noise label. For example, the residual noise label is applied by subjective assessment with respect to the corresponding electroencephalography signal.

<Configuration Example of Body Movement Noise Identification Unit 21>

Fig. 3 shows a configuration example of the body movement noise identification unit 21.

The body movement noise identification unit 21 includes an analysis window 101, a time feature calculation unit 102, a frequency feature calculation unit 103, a model accumulation unit 104, and a body movement noise label estimation unit 105.

The analysis window 101 cuts out an electroencephalography signal supplied from the EEG 11 while shifting the analysis window interval in the time axis direction by using the window function. The analysis window 101 supplies the cut-out electroencephalography signal to the time feature calculation unit 102 and the frequency feature calculation unit 103.

The time feature calculation unit 102 calculates a time feature of the electroencephalography signal, the time feature being the same type as the time feature calculated by the time feature calculation unit 63 of the learning apparatus 51. The time feature calculation unit 102 supplies the time feature of the electroencephalography signal to the body movement noise label estimation unit 105.

The frequency feature calculation unit 103 calculates a time feature of the electroencephalography signal, the time feature being the same type as the time feature calculated by the frequency feature calculation unit 64 of the learning apparatus 51. The frequency feature calculation unit 103 supplies the frequency feature of the electroencephalography signal to the body movement noise label estimation unit 105.

The model accumulation unit 104 accumulates the body movement noise identification models learned by the learning apparatus 51.

The body movement noise label estimation unit 105 estimates a body movement noise label with respect to the electroencephalography signal on the basis of the temporal feature and the frequency feature of the electroencephalography signal by using the body movement noise identification models accumulated in the model accumulation unit 104. That is, the body movement noise label estimation unit 105 estimates the presence/absence and the type of body movement noise contained in the electroencephalography signal. The body movement noise label estimation unit 105 supplies body movement noise information including an estimation result for the body movement noise label to the noise reduction processing control unit 24.

<Configuration Example of Residual Noise Identification Unit 23>

Fig. 4 shows a configuration example of the residual noise identification unit 23.

The residual noise identification unit 23 includes an analysis window 121, a time feature calculation unit 122, a frequency feature calculation unit 123, a model accumulation unit 124, and a residual noise label estimation unit 125.

The analysis window 121 cuts out the electroencephalography signal supplied from the noise reduction processing unit 22 while shifting the analysis window interval in the time axis direction by using the window function. The analysis window 121 supplies the cut-out electroencephalography signal to the time feature calculation unit 122 and the frequency feature calculation unit 123.

The time feature calculation unit 122 calculates a time feature of the electroencephalography signal, the time feature being the same type as the time feature calculated by the time feature calculation unit 63 of the learning apparatus 51. The time feature calculation unit 122 supplies the time feature of the electroencephalography signal to the residual noise label estimation unit 125.

The frequency feature calculation unit 123 calculates a time feature of the electroencephalography signal, the time feature being the same type as the time feature calculated by the frequency feature calculation unit 64 of the learning apparatus 51. The frequency feature calculation unit 123 supplies the frequency feature of the electroencephalography signal to the residual noise label estimation unit 125.

The model accumulation unit 124 accumulates the residual noise identification model learned by the learning apparatus 51.

The residual noise label estimation unit 125 estimates a residual noise label with respect to the electroencephalography signal on the basis of the temporal feature and the frequency feature of the electroencephalography signal by using the residual noise identification models accumulated in the model accumulation unit 124. That is, the residual noise label estimation unit 125 estimates the presence/absence and the type of residual noise contained in the electroencephalography signal after the noise reduction processing. The residual noise label estimation unit 125 supplies body movement noise information including an estimation result for the residual noise label to the noise reduction processing control unit 24 and the rhythmic component power correction unit 26.

<First Embodiment of Noise Reduction Processing Unit 22>

Fig. 5 shows a configuration example of a noise reduction processing unit 22a that is a first embodiment of the noise reduction processing unit 22.

The noise reduction processing unit 22a reduces contact impedance change noise contained in the electroencephalography signal. The contact impedance change noise is generated when contact impedance between the user's skin and the electrodes of the EEG 11 changes because of a body movement such as a change in facial expression during communication or video viewing in the user's daily life, for example. The contact impedance change noise appears as impulse noise including a sharp potential change or a transient phenomenon, for example.

Moreover, the noise reduction processing unit 22a in Fig. 5 reduces the contact impedance change noise by singular spectrum analysis. The singular spectrum analysis is a data-driven method of analyzing the structure itself of the electroencephalography signal without assuming a particular basic function and the like. The singular spectrum analysis is capable of separating unsteady signals like the contact impedance change noise.

The noise reduction processing unit 22a includes a trajectory matrix generation unit 151, a singular value decomposition unit 152, a noise component removal unit 153, and a recomposition unit 154.

The trajectory matrix generation unit 151 generates a trajectory matrix on the basis of the electroencephalography signal supplied from the EEG 11. The trajectory matrix generation unit 151 supplies the generated trajectory matrix to the singular value decomposition unit 152.

The singular value decomposition unit 152 decomposes the trajectory matrix into a plurality of singular vector groups by performing singular value decomposition on the trajectory matrix. The singular value decomposition unit 152 supplies information indicating a result obtained by performing the singular value decomposition on the trajectory matrix to the noise component removal unit 153. Moreover, the singular value decomposition unit 152 supplies singular values of the trajectory matrix obtained by the singular value decomposition to the noise reduction processing control unit 24.

The noise reduction processing control unit 24 sets, on the basis of the singular values of the trajectory matrix, a contact impedance change noise discrimination threshold used for discriminating components of the contact impedance change noise (hereinafter, also simply referred to as noise components) as a discrimination parameter. The noise reduction processing control unit 24 supplies the contact impedance change noise discrimination threshold to the noise component removal unit 153.

The noise component removal unit 153 removes, on the basis of the contact impedance change noise discrimination threshold, singular vectors corresponding to the noise components from the singular vector groups of the trajectory matrix. The noise component removal unit 153 supplies a singular vector groups remaining without being removed to the recomposition unit 154.

The recomposition unit 154 recomposes an electroencephalography signal on the basis of the singular vector group supplied from the noise component removal unit 153. The recomposition unit 154 supplies the recomposed electroencephalography signal to the residual noise identification unit 23 and the rhythmic component power calculation unit 25.

<Signal Processing>

Next, signal processing executed by the wearable electroencephalograph 1 will be described with reference to the flowchart in Fig. 6.

For example, the processing is started when the wearable electroencephalograph 1 is powered on and the processing is terminated when the wearable electroencephalograph 1 is powered off.

It should be noted that for example when the wearable electroencephalograph 1 is powered on, the EEG 11 starts processing of measuring an electroencephalogram of the user and supplying an electroencephalography signal to the analysis window 62 of the body movement noise identification unit 21 and the noise reduction processing unit 22. For example, when the wearable electroencephalograph 1 is powered off, the EEG 11 terminates the processing of measuring the electroencephalogram of the user and supplying an electroencephalography signal to the analysis window 62 of the body movement noise identification unit 21 and the noise reduction processing unit 22.

In Step S1, the body movement noise identification unit 21 identifies body movement noise.

Specifically, the analysis window 101 of the body movement noise identification unit 21 cuts out the electroencephalography signal supplied from the EEG 11 while shifting the analysis window interval in the time axis direction by using the window function. The analysis window 101 supplies the cut-out electroencephalography signal to the time feature calculation unit 102 and the frequency feature calculation unit 103.

The time feature calculation unit 102 calculates a predetermined time feature of the electroencephalography signal and supplies the calculated predetermined time feature to the body movement noise label estimation unit 105.

The frequency feature calculation unit 103 calculates a predetermined frequency feature of the electroencephalography signal and supplies the calculated predetermined frequency feature to the body movement noise label estimation unit 105.

The body movement noise label estimation unit 105 estimates a body movement noise label with respect to the electroencephalography signal on the basis of the temporal feature and the frequency feature of the electroencephalography signal by using the body movement noise identification models accumulated in the model accumulation unit 104. The body movement noise label estimation unit 105 supplies body movement noise information including an estimation result for the body movement noise label to the noise reduction processing control unit 24.

In Step S2, the signal processing unit 12 executes noise reduction processing. Although details of the noise reduction processing will be described later with reference to Fig. 7, the noise reduction processing unit 22 performs noise reduction processing on the electroencephalography signal supplied from the EEG 11 under the control of the noise reduction processing control unit 24. Then, the noise reduction processing unit 22 supplies the electroencephalography signal after the noise reduction processing to the residual noise identification unit 23 and the rhythmic component power calculation unit 25.

In Step S3, the residual noise identification unit 23 identifies residual noise.

Specifically, the analysis window 121 of the residual noise identification unit 23 cuts out the electroencephalography signal supplied from the noise reduction processing unit 22 while shifting the analysis window interval in the time axis direction by using the window function. The analysis window 121 supplies the cut-out electroencephalography signal to the time feature calculation unit 122 and the frequency feature calculation unit 123.

The time feature calculation unit 122 calculates a predetermined time feature of the electroencephalography signal and supplies the calculated predetermined time feature to the residual noise label estimation unit 125.

The frequency feature calculation unit 123 calculates a predetermined frequency feature of the electroencephalography signal and supplies the calculated predetermined frequency feature to the residual noise label estimation unit 125.

The residual noise label estimation unit 125 estimates a residual noise label with respect to the electroencephalography signal on the basis of the temporal feature and the frequency feature of the electroencephalography signal by using the residual noise identification models accumulated in the model accumulation unit 124. The residual noise label estimation unit 125 supplies residual noise information including an estimation result for the residual noise label to the noise reduction processing control unit 24 and the rhythmic component power correction unit 26.

In Step S4, the rhythmic component power calculation unit 25 calculates rhythmic component power. For example, on the basis of power spectrum associated with short-time Fourier transform or spectral density estimation associated with Welch's method, the rhythmic component power calculation unit 25 calculates power or the like of θ waves (4 to 7 Hz), α waves (8 to 13 Hz), and β waves (14 to 30 Hz) of the electroencephalography signal as the rhythmic component power. The rhythmic component power calculation unit 25 supplies rhythmic component power information indicating a calculation result for the rhythmic component power to the rhythmic component power correction unit 26.

In Step S5, the rhythmic component power correction unit 26 corrects the rhythmic component power.

For example, the rhythmic component power correction unit 26 invalidates the calculation result for the rhythmic component power in an interval (hereinafter, referred to as a noise remaining interval) including the contact impedance change noise as residual noise on the basis of the residual noise information. Accordingly, the rhythmic component power in the noise remaining interval is lost.

The rhythmic component power correction unit 26 interpolates the rhythmic component power in the noise remaining interval. That is, the rhythmic component power correction unit 26 interpolates the rhythmic component power in the noise remaining interval on the basis of a calculation result for rhythmic component power components in a (past and future) interval with no residual noise (hereinafter, referred to as a non-noise remaining interval) immediately temporally preceding and following the noise remaining interval. Here, simply, linear interpolation, polynomial interpolation, or the like is applied because the rhythmic component power has a high temporal correlation with no sudden change.

It should be noted that the rhythmic component power in the noise remaining interval interpolated by the rhythmic component power correction unit 26 decreases in reliability as compared to the rhythmic component power in the non-noise remaining interval calculated by the rhythmic component power calculation unit 25. In this regard, the rhythmic component power correction unit 26 sets the reliability for the rhythmic component power in the noise remaining interval to be a value lower than the reliability for the rhythmic component power in the non-noise remaining interval. For example, the rhythmic component power correction unit 26 sets the reliability for the rhythmic component power in the non-noise remaining interval to be 1.0 and sets the reliability for the rhythmic component power in the noise remaining interval to be 0.5.

It should be noted that for example the rhythmic component power correction unit 26 may interpolate the rhythmic component power in the noise remaining interval on the basis of a calculation result for rhythmic component power components of one of the non-noise remaining interval immediately temporally preceding the noise remaining interval and the non-noise remaining interval immediately following the noise remaining interval.

The rhythmic component power correction unit 26 generates output information including the rhythmic component power and the reliability and outputs the generated output information to the subsequent stage.

Then, the processing returns to Step S1 and the processing of Steps S1 to S5 is repeatedly executed.

<Details of Noise Reduction Processing>

Next, details of the noise reduction processing in Step S2 in Fig. 5 will be described with reference to the flowchart in Fig. 7.

In Step S51, the trajectory matrix generation unit 151 generates a trajectory matrix.

Specifically, as shown in Formula (1) below, the trajectory matrix generation unit 151 generates a trajectory matrix X constituted by partial time-series signals cut out from the electroencephalography signal supplied from the EEG 11.

[Expression 1]

$$X = \begin{pmatrix} x_1 & x_2 & \cdots & x_n \\ x_2 & x_3 & \cdots & x_{n+1} \\ \vdots & \vdots & \ddots & \vdots \\ x_{m-n+1} & x_{m-n+2} & \cdots & x_m \end{pmatrix} \quad \cdots (1)$$

Where $x_i$ (i = 1, 2, ..., m) indicates samples of the electroencephalography signal (observation signal) and m - n + 1 indicates an analysis window interval. It should be noted that n takes a sufficiently large value so that characteristics of the electroencephalography signal (observation signal) can be reflected.

The trajectory matrix generation unit 151 supplies the generated trajectory matrix X to the singular value decomposition unit 152.

In Step S52, the singular value decomposition unit 152 performs singular value decomposition. Specifically, the singular value decomposition unit 152 applies the singular value decomposition to the trajectory matrix X. Accordingly, as shown in Formulae (2) to (5), the trajectory matrix X is subjected to spectral decomposition using a diagonal matrix W, a matrix U, and a matrix V.

[Expression 2]

$$X = UWV^T \quad \cdots (2)$$

$$W = \begin{pmatrix} \lambda_1 & 0 & \cdots & 0 \\ 0 & \lambda_2 & \cdots & 0 \\ \vdots & \vdots & \ddots & \vdots \\ 0 & 0 & \cdots & \lambda_r \end{pmatrix} \quad \cdots (3)$$

$$U = (u_1, u_2, \cdots, u_r) \quad \cdots (4)$$

$$V = (v_1, v_2, \cdots, v_r) \quad \cdots (5)$$

The matrix U and the matrix V are orthogonal matrices satisfying $U^TU = V^TV = E$.

Diagonal components $\lambda_i$ of the diagonal matrix W are called singular values and represent variance of the decomposed components. For example, the singular values of components with large variance increase like the contact impedance change noise (impulse noise). Moreover, $\lambda_i \geq \lambda_2 \geq \ldots \geq \lambda_r$ is established.

Formula (6) below is an expression obtained by solving Formula (2).

$$X = \lambda_1 u_1 v_1^T + \lambda_2 u_2 v_2^T + \ldots + \lambda_r u_r v_r^T \quad \ldots (6)$$

Here, assuming that an i-th component in Formula (6) is $X_i = \lambda_i U_i V_i^T$ ($= \lambda_i u_i v_i^T$), the trajectory matrix X is decomposed into singular vectors $X_i$ and it is represented by a polynomial expression for the singular vectors $X_i$ as shown in Formula (7) below.

$$X = X_1 + X_2 + \ldots + X_r \quad \ldots (7)$$

The singular value decomposition unit 152 supplies information indicating a result obtained by performing the singular value decomposition on the trajectory matrix X to the noise component removal unit 153. Moreover, the singular value decomposition unit 152 supplies information indicating singular values obtained by the singular value decomposition to the noise reduction processing control unit 24.

In Step S53, the signal processing unit 12 removes noise components.

Specifically, the noise reduction processing control unit 24 estimates an upper limit for the singular values of the electroencephalography signal with no noise (hereinafter, referred to as a maximum singular value) on the basis of the singular values obtained by analyzing (performing the singular value decomposition on) the electroencephalography signal in an interval (hereinafter, referred to as a noiseless interval) estimated to contain no noise by the body movement noise identification unit 21. For example, the noise reduction processing control unit 24 estimates a maximum singular value on the basis of a cumulative density function of a singular value distribution in the past noiseless interval.

The noise reduction processing control unit 24 sets a contact impedance change noise discrimination threshold on the basis of the estimated maximum singular value. For example, the noise reduction processing unit 22 sets the estimated maximum singular value as the contact impedance change noise discrimination threshold. The noise reduction processing unit 22 supplies the set contact impedance change noise discrimination threshold to the noise component removal unit 153.

It should be noted that, for example, the noise reduction processing control unit 24 may perform setting processing for the contact impedance change noise discrimination threshold in advance or may perform the setting processing as appropriate when the noiseless interval is generated.

The noise component removal unit 153 removes noise components on the basis of the contact impedance change noise discrimination threshold. Specifically, the noise component removal unit 153 groups singular vectors $X_p$, $X_{p+1}, \ldots, X_r$ of the singular vectors $X_i$ according to Formula (7), whose singular values $\lambda_i$ are equal to or smaller than the contact impedance change noise discrimination threshold. Then, the noise component removal unit 153 removes singular vectors other than the grouped singular vectors of the respective singular vectors $X_i$ according to Formula (7). Accordingly, a matrix $X_{(p)}$ from which the noise components have been removed is generated as shown in Formula (8) below.
[Expression 3]

$$X_{(p)} \simeq X_p + X_{p+1} + \cdots X_r \qquad \cdots (8)$$

The noise component removal unit 153 supplies the matrix $X_{(p)}$ to the recomposition unit 154.
In Step S54, the recomposition unit 154 recomposes an electroencephalography signal. Specifically, the recomposition unit 154 recomposes a trajectory matrix $X_{(p)}$ according to Formula (9) below on the basis of the polynomial expression for the singular vectors according to Formula (8).
[Expression 4]

$$X_{(p)} = \begin{pmatrix} x_1' & x_2' & \cdots & x_n' \\ x_2' & x_3' & \cdots & x_{n+1}' \\ \vdots & \vdots & \ddots & \vdots \\ x_{m-n+1}' & x_{m-n+2}' & \cdots & x_m' \end{pmatrix} \qquad \cdots (9)$$

The recomposition unit 154 averages the diagonal components on the right side in Formula (9) as shown as the arrows.
In this manner, electroencephalography signals $x_1', x_2', \ldots, x_m'$ after the noise components have been removed from the electroencephalography signals $x_1, x_2, \ldots, x_m$ are generated.
The recomposition unit 154 supplies the electroencephalography signals $x_1', x_2', \ldots, x_m'$ from which the noise components have been removed to the residual noise identification unit 23 and the rhythmic component power calculation unit 25.
Here, when the contact impedance change noise (impulse noise) is not decomposed into a single component but it is decomposed into a plurality of components, the singular values $\lambda_i$ corresponding to the contact impedance change noise may decrease. Therefore, in the noise reduction processing using the contact impedance change noise discrimination threshold set on the basis of an identification result for the body movement noise identification unit 21, the contact impedance change noise may remain without being removed from the electroencephalography signal.
In this regard, the noise reduction processing control unit 24 reduces the contact impedance change noise discrimination threshold in a case where the residual noise identification unit 23 estimates that the contact impedance change noise to remain. Accordingly, components of the contact impedance change noise decomposed into the plurality of components are easily removed.
Then, the noise reduction processing ends.
In the above-mentioned manner, influences of body movement noise on an electroencephalography signal can be reduced.
That is, the accuracy of reducing the contact impedance change noise in the electroencephalography signal can be improved. For example, the contact impedance change noise discrimination threshold is set on the basis of the identification result for the body movement noise, and components of the contact impedance change noise are removed. Therefore, the accuracy of reducing the contact impedance change noise in the electroencephalography signal is improved. Moreover, the contact impedance change noise discrimination threshold is corrected on the basis of the identification result for the residual noise, and components of the contact impedance change noise are removed. Therefore, the accuracy of reducing the contact impedance change noise in the electroencephalography signal is further improved.
In addition, on the basis of the identification result for the residual noise, the rhythmic component power is corrected and the reliability for the rhythmic component power is set. Accordingly, the occurrence of a failure due to body movement noise can be suppressed in an apparatus or application using rhythmic component power at the subsequent stage.

<<2. Second Embodiment>>

Next, a second embodiment of the present technology will be described with reference to Figs. 8 and 9.
The second embodiment differs from the first embodiment in terms of the type of body movement noise as a target to be reduced. Specifically, in the second embodiment, reduction processing for electromyography noise generated by a change in the user's facial expression, chewing, conversation, a neck motion, and the like is performed.

<Second Embodiment of Noise Reduction Processing Unit 22>

The second embodiment differs from the first embodiment in terms of the configuration of the noise reduction processing unit 22 of the wearable electroencephalograph 1. Fig. 8 shows a configuration example of a noise reduction processing unit 22b which is a second embodiment of the noise reduction processing unit 22.

The noise reduction processing unit 22b reduces electromyography noise contained in the electroencephalography signal by using empirical mode decomposition.

A noise reduction processing unit 22b includes an empirical mode decomposition unit 201, a delay unit 202, a canonical correlation analysis unit 203, a noise component removal unit 204, an inverse canonical correlation analysis unit 205, and an inverse empirical mode decomposition unit 206.

The empirical mode decomposition unit 201 performs empirical mode decomposition on the electroencephalography signal supplied from the EEG 11 and decomposes the electroencephalography signal into a plurality of singular mode functions. The empirical mode decomposition unit 201 supplies the respective singular mode functions to the delay unit 202 and the canonical correlation analysis unit 203.

The delay unit 202 delays (shifts) the respective singular mode functions by a predetermined time and supplies the delayed singular mode function to the canonical correlation analysis unit 203.

For each the respective singular mode function, the canonical correlation analysis unit 203 performs canonical correlation analysis for the original singular mode function before the delay and the delayed singular mode function. The canonical correlation analysis unit 203 supplies information indicating canonical correlation analysis results for the respective singular mode functions to the noise component removal unit 204. The canonical correlation analysis unit 203 supplies autocorrelation values of the respective singular mode functions before and after the delay (autocorrelation values in a time direction of the respective singular mode functions) to the noise reduction processing control unit 24.

The noise reduction processing control unit 24 sets, on the basis of the autocorrelation values in the time direction of the respective singular mode functions, an electromyography noise discrimination threshold used for discriminating components of the electromyography noise (hereinafter, also simply referred to as noise components) as a discrimination parameter. The noise reduction processing control unit 24 supplies the electromyography noise discrimination threshold to the noise component removal unit 204.

The noise component removal unit 204 removes singular mode functions corresponding to the noise components on the basis of the electromyography noise discrimination threshold. The noise component removal unit 204 supplies singular mode functions remaining without being removed to the inverse canonical correlation analysis unit 205.

The inverse canonical correlation analysis unit 205 performs inverse canonical correlation analysis with respect to the respective singular mode functions remaining without being removed. The inverse canonical correlation analysis unit 205 supplies the respective singular mode functions after the inverse canonical correlation analysis to the inverse empirical mode decomposition unit 206.

The inverse empirical mode decomposition unit 206 restores the electroencephalography signal by performing inverse empirical mode decomposition with on the respective singular mode functions after the inverse canonical correlation analysis. The inverse empirical mode decomposition unit 206 supplies the restored electroencephalography signal to the residual noise identification unit 23 and the rhythmic component power calculation unit 25.

<Noise Reduction Processing>

In the second embodiment, the signal processing is executed in accordance with the flowchart in Fig. 6 as in the first embodiment.

It should be noted that in the second embodiment, in Step S5, the rhythmic component power in the interval (noise remaining interval) containing the electromyography noise as the residual noise is corrected.

Moreover, the second embodiment differs from the first embodiment in terms of the contents of the noise reduction processing in Step S2.

Here, details of the noise reduction processing in the second embodiment will be described with reference to the flowchart in Fig. 9.

In Step S101, the empirical mode decomposition unit 201 performs empirical mode decomposition. Specifically, the empirical mode decomposition unit 201 decomposes the electroencephalography signal in one channel into a plurality of singular mode functions by using the empirical mode decomposition. The empirical mode decomposition unit 201 supplies the respective singular mode functions to the delay unit 202 and the canonical correlation analysis unit 203.

In Step S102, the delay unit 202 delays the respective singular mode functions by a time corresponding to one sample of the electroencephalography signal. The delay unit 202 supplies the delayed respective singular mode functions to the canonical correlation analysis unit 203.

In Step S103, the canonical correlation analysis unit 203 performs canonical correlation analysis. Specifically, for each singular mode function, the canonical correlation analysis unit 203 performs canonical correlation analysis for the original singular mode function before the delay and the delayed singular mode function. Accordingly, the original singular mode function and the delayed singular mode function are linearly transformed, and a plurality of common signal components between both is calculated in order from the highest autocorrelation. The canonical correlation analysis unit 203 supplies information indicating canonical correlation analysis results for the respective singular mode functions to the noise component removal unit 204. Moreover, the canonical correlation analysis unit 203 supplies autocorrelation values for the common signal components to the noise reduction processing control unit 24.

In Step S104, the signal processing unit 12 removes noise components.

Specifically, the noise reduction processing control unit 24 sets the electromyography noise discrimination threshold on the basis of the autocorrelation values for the common signal components. For example, the noise reduction processing unit 22 estimates a minimum value (hereinafter, referred to as a minimum autocorrelation value) of the autocorrelation values of the canonical correlation analysis for the singular mode functions of the electroencephalography signal with no noise on the basis of the analysis result (autocorrelation values for the common signal components) for the electroencephalography signal in the noiseless interval estimated to contain no noise by the body movement noise identification unit 21.

The noise reduction processing control unit 24 sets the electromyography noise discrimination threshold on the basis of the estimated minimum autocorrelation value. For example, the noise reduction processing control unit 24 sets the estimated minimum autocorrelation value as the electromyography noise discrimination threshold. The noise reduction processing unit 22 supplies the set electromyography noise discrimination threshold to the noise component removal unit 153.

It should be noted that, for example, the noise reduction processing control unit 24 may perform setting processing for the electromyography noise discrimination threshold in advance or may perform the setting processing as appropriate when the noiseless interval is generated.

The noise reduction processing control unit 24 supplies the electromyography noise discrimination threshold to the noise component removal unit 204.

The noise component removal unit 204 removes noise components on the basis of the autocorrelation values of the canonical correlation analysis for the respective singular mode functions and the electromyography noise discrimination threshold.

Here, the electromyography noise has characteristics close to white noise and has low autocorrelation in the time direction. Thus, in singular mode functions corresponding to the electromyography noise, the autocorrelation values for the common signal components between the original singular mode function and the delayed singular mode function decrease. On the other hand, the electroencephalography signal has a high autocorrelation in the time direction. Thus, in singular mode functions corresponding to the electroencephalography signal, the autocorrelation values for the common signal components between the original singular mode function and the delayed singular mode function increase.

In this regard, the noise component removal unit 204 determines that common signal components whose autocorrelation values are equal to or larger than the electromyography noise discrimination threshold are electroencephalography signal components. On the other hand, the noise component removal unit 204 determines that common signal components whose autocorrelation values are smaller than the electromyography noise discrimination threshold are noise components.

The noise component removal unit 204 supplies singular mode functions (linearly transformed singular mode functions) corresponding to the electroencephalography signal to the inverse canonical correlation analysis unit 205. Accordingly, singular mode functions corresponding to the electromyography noise are removed.

In Step S105, the inverse canonical correlation analysis unit 205 performs inverse canonical correlation analysis. Specifically, the inverse canonical correlation analysis unit 205 restores the singular mode functions before the canonical correlation analysis by performing inverse linear transformation on the singular mode functions (linearly transformed singular mode functions) determined to correspond to the electroencephalography signal. The inverse canonical correlation analysis unit 205 supplies the restored singular mode function to an inverse empirical mode decomposition unit 208.

In Step S106, the inverse empirical mode decomposition unit 208 performs inverse empirical mode conversion. Specifically, the inverse empirical mode decomposition unit 208 restores the electroencephalography signal by recomposing (adding up) the restored singular mode functions.

Accordingly, the electroencephalography signal is restored on the basis of the singular mode function from which singular mode functions corresponding to the electromyography noise have been removed, and the electromyography noise in the electroencephalography signal is reduced.

The inverse empirical mode decomposition unit 208 supplies the restored electroencephalography signal to the residual noise identification unit 23 and the rhythmic component power calculation unit 25.

Here, the electromyography noise may be decomposed into a plurality of singular mode functions due to the mode mixing problem in the empirical mode decomposition. Therefore, it is difficult to set the electromyography noise discrimination threshold, and the electromyography noise may remain in the electroencephalography signal after the noise reduction processing.

In this regard, the noise reduction processing unit 22 corrects the electromyography noise discrimination threshold, for example, on the basis of the identification result for the residual noise by the residual noise identification unit 23. Specifically, for example, the noise reduction processing unit 22 increases the electromyography noise discrimination threshold in a case where the residual noise identification unit 23 estimates that the electromyography noise remains in the electroencephalography signal. Accordingly, the electromyography noise decomposed into the plurality of singular mode functions is easily removed, and the accuracy for the noise reduction processing is improved.

It should be noted that the noise reduction processing unit 22 may recompose the singular mode functions by inverse linear transformation for example after the noise reduction processing unit 22 performs identification processing for the body movement noise with respect to the respective components in the canonical correlation analysis and discards components containing the electromyography noise (after replacing the components by 0).

Alternatively, for example, in a case where the rhythmic component frequency range for the electroencephalography signal is set to be within a $\beta$ frequency range, the noise reduction processing unit 22 may apply a low pass filter for attenuating components beyond the $\beta$ frequency range to the respective components in the canonical correlation analysis without discarding the components containing the electromyography noise. Then, the noise reduction processing unit 22 may inversely linearly transform the components to which the low pass filter is applied and recompose the singular mode functions.

It should be noted that the contact impedance change noise and the electromyography noise for example due to a facial expression change are simultaneously superimposed on the electroencephalography signal. Thus, for example, the first embodiment and the second embodiment can also be combined. That is, the noise reduction processing unit 22 of the wearable electroencephalograph 1 may include both configurations of the noise reduction processing unit 22a in Fig. 5 and the noise reduction processing unit 22b in Fig. 8 for reducing both the contact impedance change noise and the electromyography noise.

## <<3. Third Embodiment>>

Next, a third embodiment of the present technology will be described with reference to Figs. 10 to 12.

### <Configuration Example of Wearable Electroencephalograph 301>

Fig. 10 shows a configuration example of a wearable electroencephalograph 301 which is a second embodiment of the wearable electroencephalograph to which the present technology is applied. It should be noted that in the figure, portions corresponding to those of the wearable electroencephalograph 1 in Fig. 1 are denoted by the same reference signs and the descriptions are omitted as appropriate.

The wearable electroencephalograph 301 is the same as the wearable electroencephalograph 1 in terms of the fact that the wearable electroencephalograph 301 includes the EEG 11. The wearable electroencephalograph 301 differs from the wearable electroencephalograph 1 in terms of the fact that the wearable electroencephalograph 301 includes a signal processing unit 312 instead of the signal processing unit 12 and additionally includes an IMU 311. The signal processing unit 312 is the same as the signal processing unit 12 in terms of the fact that the signal processing unit 312 includes the body movement noise identification unit 21, the noise reduction processing unit 22, the residual noise identification unit 23, and the rhythmic component power calculation unit 25. On the other hand, the signal processing unit 312 differs from the signal processing unit 12 in terms of the fact that the signal processing unit 312 includes a noise reduction processing control unit 322 and a rhythmic component power correction unit 323 instead of the noise reduction processing control unit 24 and the rhythmic component power correction unit 26 and additionally includes a body movement analysis unit 321.

The IMU 311 includes an acceleration sensor and a gyro sensor and measures acceleration and angular velocity generated by a body movement of the user. The IMU 311 supplies an acceleration signal indicating a measurement result for acceleration and a gyro signal indicating a measurement result for angular velocity to the body movement analysis unit 321.

The body movement analysis unit 321 analyzes the body movement of the user on the basis of the acceleration signal and gyro signal. For example, the body movement analysis unit 321 analyzes the body movement of the user to thereby identify a context of the body movement of the user (hereinafter, referred to as a body movement context), and calculates features relating to the identified body movement context.

The body movement context is classified as, for example, a rest state in which the head does not move, a state in which the head is tilted, a state in which repetitive movements such as walking or running occur, or the like.

For example, an identification model learned in advance is used for identifying the body movement context.

For example, learning data constituted by a dataset group including input data and training data is used for the learning of the identification model. For example, in a case where the user's state is a predetermined body movement context, the input data includes the acceleration signal and gyro signal obtained by the IMU sensor 311. The training data includes a body movement context label indicating the body movement context corresponding to the input data. The body movement context label is classified as, for example, 0: rest state, 1: state in which the head is tilted, 2: state in which repetitive movements such as walking or running occur, and 3: other state.

It should be noted that for example the body movement analysis unit 321 may identify a body movement context of the user on the basis of the acceleration signal and gyro signal from the IMU 12 without using the identification model.

Moreover, the body movement analysis unit 321 calculates features relating to the identified body movement context on the basis of the acceleration signal and gyro signal. The types of features relating to the body movement context to be calculated and the number of features are not particularly limited. For example, features such as velocity, acceleration, angular velocity, movement direction, and amount of movement are calculated.

The body movement analysis unit 321 supplies body movement information including an identification result for the body movement context of the user and a calculation result for the features to the noise reduction processing control unit 322 and the rhythmic component power correction unit 323.

The noise reduction processing control unit 322 acquires body movement noise information from the body movement noise identification unit 21, acquires analysis information from the noise reduction processing unit 22, and acquires residual noise information from the residual noise identification unit 23. The noise reduction processing control unit 322 controls the noise reduction processing by the noise reduction processing unit 22 on the basis of at least one of the body movement noise information, the residual noise information, the body movement information, and the analysis information.

The rhythmic component power correction unit 323 corrects the rhythmic component power on the basis of the residual noise information and the body movement information and sets the reliability for the rhythmic component power. The rhythmic component power correction unit 26 outputs output information including the corrected rhythmic component power and the reliability.

<First Embodiment of Signal Processing>

Next, a first embodiment of the signal processing executed by the wearable electroencephalograph 301 will be described with reference to the flowchart in Fig. 11.

For example, the processing is started when the wearable electroencephalograph 301 is powered on and the processing is terminated when the wearable electroencephalograph 301 is powered off.

It should be noted that for example when the wearable electroencephalograph 301 is powered on, the EEG 11 starts processing of measuring an electroencephalogram of the user and supplying an electroencephalography signal to the analysis window 62 of the body movement noise identification unit 21 and the noise reduction processing unit 22. Moreover, the IMU 12 starts processing of measuring acceleration and angular velocity of the user and supplying acceleration signal and gyro signal to the body movement analysis unit 321.

For example, when the wearable electroencephalograph 301 is powered off, the EEG 11 terminates the processing of measuring the electroencephalogram of the user and supplying an electroencephalography signal to the analysis window 62 of the body movement noise identification unit 21 and the noise reduction processing unit 22. Moreover, the IMU 12 terminates the processing of measuring the acceleration and angular velocity of the user and supplying the acceleration signal and gyro signal to the body movement analysis unit 321.

In Step S201, the body movement analysis unit 321 analyzes a body movement of the user on the basis of the acceleration signal and gyro signal from the IMU 12. For example, the body movement analysis unit 321 analyzes the body movement of the user to thereby identify a body movement context of the user, and calculates features relating to the identified body movement context. The body movement analysis unit 321 supplies body movement information including an identification result for the body movement context of the user and a calculation result for the features to the noise reduction processing control unit 322 and the rhythmic component power correction unit 323.

In Step S202, body movement noise is identified as in the processing of Step S1 in Fig. 6.

In Step S203, the noise reduction processing is executed as in the processing of Step S2 in Fig. 6.

In Step S204, residual noise is identified as in the processing of Step S3 in Fig. 6.

In Step S205, rhythmic component power is calculated as in the processing of Step S4 in Fig. 6.

In Step S206, the rhythmic component power correction unit 26 corrects the rhythmic component power. Specifically, the rhythmic component power correction unit 26 corrects the rhythmic component power in the noise remaining interval as in the processing of Step S5 in Fig. 6.

Moreover, the rhythmic component power correction unit 26 sets the reliability for the rhythmic component power in the non-noise remaining interval to be 1.0.

In addition, the rhythmic component power correction unit 26 sets the reliability for the rhythmic component power in the noise remaining interval on the basis of the body movement information.

For example, during walking or running in a daily life, the time for which the body movement noise is mixed in the electroencephalography signal is long and the strength of the body movement noise also increases. Therefore, the interval for which the effect of the noise reduction processing is weak may increases. Thus, the accuracy for the rhythmic component power in a case of correcting the rhythmic component power on the basis of the (past/future) non-noise remaining interval immediately temporally preceding or following the noise remaining interval lowers.

In this regard, for example, body movement contexts in which the performance of the noise reduction processing lowers are known by previous experiments, tests, and the like. In a case where the body movement context in which the performance of the noise reduction processing lowers is recognized by the body movement analysis unit 321, the rhythmic component power correction unit 26 sets the reliability for the rhythmic component power in the noise remaining interval to be 0. On the other hand, in a case where the body movement context in which the performance of the noise reduction processing lowers is not recognized by the body movement analysis unit 321, the rhythmic component power correction unit 26 sets the reliability for the rhythmic component power in the noise remaining interval to be 0.5.

In this manner, the reliability for the rhythmic component power is more suitably set on the basis of an analysis result for the body movement of the user. Accordingly, in an apparatus or application using the rhythmic component power at the subsequent stage, generation of a failure due to residual noise can be more suitably suppressed.

Then, the processing returns to Step S201 and the processing of Steps S201 to S206 is repeatedly executed.

<Second Embodiment of Signal Processing>

Next, a second embodiment of the signal processing executed by the wearable electroencephalograph 301 will be described with reference to the flowchart in Fig. 12.

For example, the processing is started when the wearable electroencephalograph 301 is powered on and the processing is terminated when the wearable electroencephalograph 301 is powered off.

It should be noted that for example when the wearable electroencephalograph 301 is powered on, the EEG 11 starts processing of measuring an electroencephalogram of the user and supplying an electroencephalography signal to the analysis window 62 of the body movement noise identification unit 21 and the noise reduction processing unit 22. Moreover, the IMU 12 starts processing of measuring acceleration and angular velocity of the user and supplying acceleration signal and gyro signal to the body movement analysis unit 321.

For example, when the wearable electroencephalograph 301 is powered off, the EEG 11 terminates the processing of measuring the electroencephalogram of the user and supplying an electroencephalography signal to the analysis window 62 of the body movement noise identification unit 21 and the noise reduction processing unit 22. Moreover, the IMU 12 terminates the processing of measuring the acceleration and angular velocity of the user and supplying the acceleration signal and gyro signal to the body movement analysis unit 321.

In Step S251, the body movement of the user is analyzed as in the processing of Step S201 in Fig. 11.

In Step S252, body movement noise is identified as in the processing of Step S1 in Fig. 6.

In Step S253, the noise reduction processing control unit 322 determines whether or not the user is in a rest state on the basis of the body movement noise information and the body movement information. For example, the noise reduction processing control unit 322 determines whether or not the electroencephalography signal contains the body movement noise on the basis of the body movement noise information. Moreover, the noise reduction processing control unit 322 determines whether or not the user's head is stationary on the basis of the body movement information. The noise reduction processing control unit 322 determines that the user is not in the rest state in a case where the electroencephalography signal contains the body movement noise or the user's head is not stationary, and the processing proceeds to Step S254.

In Step S254, the noise reduction processing is executed as in the processing of Step S2 in Fig. 6.

In Step S255, residual noise is identified as in the processing of Step S3 in Fig. 6.

Then, the processing proceeds to Step S257.

On the other hand, in Step S253, the noise reduction processing control unit 322 determines that the user is in the rest state in a case where the electroencephalography signal contains no body movement noise and the user's head is stationary, and the processing proceeds to Step S256.

In Step S256, the noise reduction processing unit 22 halts the noise reduction processing under the control of the noise reduction processing control unit 322. Specifically, the noise reduction processing unit 22 supplies the electroencephalography signal supplied from the EEG 11 to the rhythmic component power calculation unit 25 as it is without performing the noise reduction processing under the control of the noise reduction processing control unit 322. Moreover, the noise reduction processing control unit 322 stops the supply of the electroencephalography signal to the residual noise identification unit 23.

Then, the processing proceeds to Step S257.

In Step S257, rhythmic component power is calculated as in the processing of Step S4 in Fig. 6.

In Step S258, the rhythmic component power is corrected as in the processing of Step S206 in Fig. 11.
Then, the processing returns to Step S251 and the processing of Steps S251 to S258 is repeatedly executed.
Accordingly, the noise reduction processing is halted in a case where it is determined that the user is in the rest state. Therefore, the power consumption for the wearable electroencephalograph 301 can be reduced.

<<4. Modified Examples>>

Hereinafter, modified examples of the above-mentioned embodiment of the present technology will be described.
In the above description, the example in which the wearable electroencephalograph measures the electroencephalography signal in one channel. However, the present technology can also be applied to a case where the wearable electroencephalograph measures electroencephalography signals in a plurality of channels. In this case, for example, the above-mentioned signal processing is executed for each channel for the electroencephalography signal.
For example, the body movement noise identification unit 21 may identify body movement noise on the basis of the body movement information by the body movement analysis unit 321 in addition to the electroencephalography signal. In this case, for example, in the learning processing for the body movement noise identification model, input data including the electroencephalography signal and the body movement information is used.
Similarly, for example, the residual noise identification unit 23 may identify the residual noise on the basis of the body movement information by the body movement analysis unit 321 in addition to the electroencephalography signal. In this case, for example, in the learning processing for the residual noise identification model, input data including the electroencephalography signal and the body movement information after noise reduction is used.
Moreover, for example, the body movement noise identification unit 21 may identify body movement noise on the basis of the acceleration signal and gyro signal by the IMU 311 in addition to the electroencephalography signal. In this case, for example, in the learning processing for the body movement noise identification model, input data including the electroencephalography signal, the acceleration signal, and the gyro signal is used.
Similarly, for example, the residual noise identification unit 23 may identify the residual noise on the basis of the acceleration signal and gyro signal by the IMU 311 in addition to the electroencephalography signal. In this case, for example, in the learning processing for the residual noise identification model, input data including the electroencephalography signal, the acceleration signal, and the gyro signal is used.
For example, the noise reduction processing unit 22 may analyze the electroencephalography signal by an analysis method other than the singular spectrum analysis method and the empirical mode decomposition for removing body movement noise components. Moreover, for example, the noise reduction processing unit 22 may analyze the electroencephalography signal by a combination of a plurality of analysis methods for removing body movement noise components.
The wearable electroencephalograph 1 does not necessarily need to be constituted by a single apparatus. For example, the EEG 11 and the signal processing unit 12 may be provided in different apparatuses. For example, the signal processing unit 12 may be constituted by a plurality of apparatuses. Specifically, for example, the noise reduction processing unit 22 and the noise reduction processing control unit 24 may be provided in different apparatuses.
Similarly, the wearable electroencephalograph 331 does not necessarily need to be constituted by a single apparatus. For example, the EEG 11, the IMU 311, and the signal processing unit 312 may be respectively provided in different apparatuses. For example, the signal processing unit 312 may be constituted by a plurality of apparatuses. Specifically, for example, the noise reduction processing unit 22 and the noise reduction processing control unit 322 may be provided in different apparatuses.
The present technology can also be applied to, for example, a system, an apparatus, an application, and the like that perform at least one of measuring the electroencephalography signal or processing the electroencephalography signal, besides the wearable electroencephalograph.

<<5. Others>>

<Configuration Example of Computer>

The above-mentioned series of processing may be executed by hardware or may be executed by software. If the series of processing is executed by software, programs that configure the software are installed in a computer. Here, the computer includes a computer incorporated in dedicated hardware, a general-purpose personal computer, for example, capable of executing various functions by installing various programs, and the like.
Fig. 13 is a block diagram showing a configuration example of hardware of the computer that executes the above-mentioned series of processing in accordance with the program.
In a computer 1000, a central processing unit (CPU) 1001, a read only memory (ROM) 1002, and a random access memory (RAM) 1003 are connected to one another through a bus 1004.

An input/output interface 1005 is also connected to the bus 1004. An input unit 1006, an output unit 1007, a storage unit 1008, a communication unit 1009, and a drive 1010 are connected to the input/output interface 1005.

The input unit 1006 includes an input switch, a button, a microphone, an image pickup element, and the like. The output unit 1007 includes a display, a loudspeaker, and the like. The storage unit 1008 includes a hard disk, a nonvolatile memory, and the like. The communication unit 1009 includes a network interface and the like. The drive 1010 drives a removable medium 1011 such as a magnetic disk, an optical disc, a magneto-optical disk, and a semiconductor memory.

In the thus configured computer 1000, the CPU 1001 loads, for example, programs stored in the storage unit 1008 into the RAM 1003 via the input/output interface 1005 and the bus 1004 and executes them. In this manner, the above-mentioned series of processing is performed.

Programs executed by the computer 1000 (CPU 1001) can be, for example, provided recorded on the removable medium 1011 that is a package medium. Moreover, the program can be provided via a wired or wireless transmission medium such as a local area network, the Internet, and digital satellite broadcasting.

In the computer 1000, the program can be installed into the storage unit 1008 via the input/output interface 1005 by mounting the removable medium 1011 on the drive 1010. Moreover, the program can be received by the communication unit 1009 via a wired or wireless transmission medium and can be installed into the storage unit 1008. Otherwise, the program can be installed into the ROM 1002 or the storage unit 1008 in advance.

It should be noted that the programs executed by the computer may be programs processed chronologically in the order described in the present specification or may be programs processed in parallel or at a required time, e.g., upon calling.

Moreover, in the present specification, the system means a set of a plurality of components (apparatuses, modules (parts), etc.) and it does not matter whether or not all the components are contained in the same casing. Therefore, a plurality of apparatuses housed in separate casings and connected via a network and a single apparatus including a plurality of modules housed in the same casing are both considered as the system.

In addition, embodiments of the present technology are not limited to the above-mentioned embodiments and various modifications can be made without departing from the gist of the present technology.

For example, the present technology can take a cloud computing configuration in which a plurality of apparatuses shares and cooperatively processes a single function via a network.

Moreover, a plurality of apparatuses can share and execute the respective steps described above with reference to the above-mentioned flowcharts rather than executing them by a single apparatus.

In addition, if a single step is constituted by a plurality of processes, a plurality of apparatuses can share and execute the plurality of processes of the single step rather than executing them by a single apparatus.

<Combination Examples of Configurations>

The present technology can also take the following configurations.

(1) An information processing apparatus, including:

a body movement noise identification unit that identifies, on the basis of an electroencephalography signal of a user, body movement noise contained in the electroencephalography signal; and
a noise reduction processing control unit that controls, on the basis of an identification result for the body movement noise, noise reduction processing of reducing the body movement noise contained in the electroencephalography signal.

(2) The information processing apparatus according to (1), further including

a residual noise identification unit that identifies, on the basis of the electroencephalography signal after the noise reduction processing, residual noise which is the body movement noise remaining in the electroencephalography signal, in which
the noise reduction processing control unit controls the noise reduction processing on the basis of the identification result for the body movement noise and an identification result for the residual noise.

(3) The information processing apparatus according to (2), further including:

a rhythmic component power calculation unit that calculates rhythmic component power of the electroencephalography signal after the noise reduction processing; and

a rhythmic component power correction unit that corrects the rhythmic component power on the basis of the identification result for the residual noise.

(4) The information processing apparatus according to (3), in which
the rhythmic component power correction unit sets reliability for the rhythmic component power on the basis of the identification result for the residual noise.
(5) The information processing apparatus according to (4), further including

a body movement analysis unit that analyzes a body movement of the user, in which
the rhythmic component power correction unit sets the reliability for the rhythmic component power on the basis of the identification result for the residual noise and an analysis result for the body movement of the user.

(6) The information processing apparatus according to (5), in which

the body movement noise identification unit identifies, on the basis of the electroencephalography signal and the analysis result for the body movement of the user, the body movement noise contained in the electroencephalography signal, and
the residual noise identification unit identifies, on the basis of the electroencephalography signal after the noise reduction processing and the analysis result for the body movement of the user, the residual noise remaining in the electroencephalography signal.

(7) The information processing apparatus according to any one of (3) to (6), in which
the rhythmic component power correction unit interpolates the rhythmic component power in a noise remaining interval in which the residual noise is estimated to remain by the residual noise identification unit, on the basis of the rhythmic component power in at least one interval of an interval immediately preceding the noise remaining interval or an interval immediately following the noise remaining interval.
(8) The information processing apparatus according to any one of (2) to (7), in which

the noise reduction processing control unit sets, on the basis of the identification result for the body movement noise and the identification result for the residual noise, a discrimination parameter used for discriminating components of the body movement noise contained in the electroencephalography signal on the basis of an analysis result for the electroencephalography signal, further including
a noise reduction processing unit that removes components of the body movement noise from the electroencephalography signal by using the discrimination parameter.

(9) The information processing apparatus according to (8), in which
the noise reduction processing control unit corrects the discrimination parameter so that components of the body movement noise are easily removed in a case where the residual noise is estimated to remain in the electroencephalography signal by the residual noise identification unit.
(10) The information processing apparatus according to (1), further including
a noise reduction processing unit that executes noise reduction processing.
(11) The information processing apparatus according to (10), in which

the noise reduction processing control unit sets, on the basis of the identification result for the body movement noise, a discrimination parameter used for discriminating components of the body movement noise contained in the electroencephalography signal on the basis of an analysis result for the electroencephalography signal, and
the noise reduction processing unit removes components of the body movement noise from the electroencephalography signal by using the discrimination parameter.

(12) The information processing apparatus according to (11), in which
the noise reduction processing control unit sets the discrimination parameter on the basis of an analysis result for the electroencephalography signal in an interval estimated not to contain the body movement noise by the body movement noise identification unit.
(13) The information processing apparatus according to (11) or (12), in which
the discrimination parameter is a threshold used for discriminating components of the body movement noise contained in the electroencephalography signal on the basis of singular values of a trajectory matrix in singular spectrum analysis based on the electroencephalography signal.
(14) The information processing apparatus according to any one of (11) to (13), in which

the discrimination parameter is a threshold used for discriminating the body movement noise contained in the electroencephalography signal on the basis of an autocorrelation value of a common component in canonical correlation analysis between a singular mode function of the electroencephalography signal and a delayed singular mode function.

(15) The information processing apparatus according to (1), further including

a body movement analysis unit that analyzes a body movement of the user, in which
the noise reduction processing control unit controls the noise reduction processing on the basis of the identification result for the body movement noise and an analysis result for the body movement of the user.

(16) The information processing apparatus according to (15), in which
the noise reduction processing control unit stops the noise reduction processing in a case of determining that the user is in a rest state on the basis of the identification result for the body movement noise and the analysis result for the body movement of the user.

(17) The information processing apparatus according to (15), in which
the body movement noise identification unit identifies, on the basis of the electroencephalography signal and the analysis result for the body movement of the user, the body movement noise contained in the electroencephalography signal.

(18) An information processing method, including:

identifying, on the basis of an electroencephalography signal of a user, body movement noise contained in the electroencephalography signal; and
controlling, on the basis of an identification result for the body movement noise, noise reduction processing of reducing the body movement noise contained in the electroencephalography signal.

(19) A program that causes a computer to execute processing of:

identifying, on the basis of an electroencephalography signal of a user, body movement noise contained in the electroencephalography signal; and
controlling, on the basis of an identification result for the body movement noise, noise reduction processing of reducing the body movement noise contained in the electroencephalography signal.

It should be noted that the effects set forth herein are merely exemplary and not limitative, and other effects may be provided.

Reference Signs List

| | |
|---|---|
| 1 | wearable electroencephalograph |
| 11 | EEG |
| 12 | signal processing unit |
| 21 | body movement noise identification unit |
| 22, 22a, 22b | noise reduction processing unit |
| 23 | residual noise identification unit |
| 24 | noise reduction processing control unit |
| 25 | rhythmic component power calculation unit |
| 26 | rhythmic component power correction unit |
| 51 | learning apparatus |
| 65 | model learning unit |
| 105 | body movement noise label estimation unit |
| 125 | residual noise label estimation unit |
| 151 | trajectory matrix generation unit |
| 152 | singular value decomposition unit |
| 153 | noise component removal unit |
| 154 | recomposition unit |
| 201 | empirical mode decomposition unit |
| 202 | delay unit |

| | |
|---|---|
| 203 | canonical correlation analysis unit |
| 204 | noise component removal unit |
| 205 | inverse canonical correlation analysis unit |
| 206 | inverse empirical mode decomposition unit |
| 301 | wearable electroencephalograph |
| 311 | IMU |
| 312 | signal processing unit |
| 321 | body movement analysis unit |
| 322 | noise reduction processing control unit |
| 323 | rhythmic component power correction unit |

## Claims

**1.** An information processing apparatus, comprising:

a body movement noise identification unit that identifies, on a basis of an electroencephalography signal of a user, body movement noise contained in the electroencephalography signal; and
a noise reduction processing control unit that controls, on a basis of an identification result for the body movement noise, noise reduction processing of reducing the body movement noise contained in the electroencephalography signal.

**2.** The information processing apparatus according to claim 1, further comprising

a residual noise identification unit that identifies, on a basis of the electroencephalography signal after the noise reduction processing, residual noise which is the body movement noise remaining in the electroencephalography signal, wherein
the noise reduction processing control unit controls the noise reduction processing on a basis of the identification result for the body movement noise and an identification result for the residual noise.

**3.** The information processing apparatus according to claim 2, further comprising:

a rhythmic component power calculation unit that calculates rhythmic component power of the electroencephalography signal after the noise reduction processing; and
a rhythmic component power correction unit that corrects the rhythmic component power on a basis of the identification result for the residual noise.

**4.** The information processing apparatus according to claim 3, wherein
the rhythmic component power correction unit sets reliability for the rhythmic component power on a basis of the identification result for the residual noise.

**5.** The information processing apparatus according to claim 4, further comprising

a body movement analysis unit that analyzes a body movement of the user, wherein
the rhythmic component power correction unit sets the reliability for the rhythmic component power on a basis of the identification result for the residual noise and an analysis result for the body movement of the user.

**6.** The information processing apparatus according to claim 5, wherein

the body movement noise identification unit identifies, on a basis of the electroencephalography signal and the analysis result for the body movement of the user, the body movement noise contained in the electroencephalography signal, and
the residual noise identification unit identifies, on a basis of the electroencephalography signal after the noise reduction processing and the analysis result for the body movement of the user, the residual noise remaining in the electroencephalography signal.

**7.** The information processing apparatus according to claim 3, wherein
the rhythmic component power correction unit interpolates the rhythmic component power in a noise remaining interval in which the residual noise is estimated to remain by the residual noise identification unit, on a basis of the rhythmic component power in at least one interval of an interval immediately preceding the noise remaining interval or an interval immediately following the noise remaining interval.

**8.** The information processing apparatus according to claim 2, wherein

the noise reduction processing control unit sets, on a basis of the identification result for the body movement noise and the identification result for the residual noise, a discrimination parameter used for discriminating components of the body movement noise contained in the electroencephalography signal on a basis of an analysis result for the electroencephalography signal, further comprising

a noise reduction processing unit that removes components of the body movement noise from the electroencephalography signal by using the discrimination parameter.

**9.** The information processing apparatus according to claim 8, wherein
the noise reduction processing control unit corrects the discrimination parameter so that components of the body movement noise are easily removed in a case where the residual noise is estimated to remain in the electroencephalography signal by the residual noise identification unit.

**10.** The information processing apparatus according to claim 1, further comprising
a noise reduction processing unit that executes noise reduction processing.

**11.** The information processing apparatus according to claim 10, wherein

the noise reduction processing control unit sets, on a basis of the identification result for the body movement noise, a discrimination parameter used for discriminating components of the body movement noise contained in the electroencephalography signal on a basis of an analysis result for the electroencephalography signal, and

the noise reduction processing unit removes components of the body movement noise from the electroencephalography signal by using the discrimination parameter.

**12.** The information processing apparatus according to claim 11, wherein
the noise reduction processing control unit sets the discrimination parameter on a basis of an analysis result for the electroencephalography signal in an interval estimated not to contain the body movement noise by the body movement noise identification unit.

**13.** The information processing apparatus according to claim 11, wherein
the discrimination parameter is a threshold used for discriminating components of the body movement noise contained in the electroencephalography signal on a basis of singular values of a trajectory matrix in singular spectrum analysis based on the electroencephalography signal.

**14.** The information processing apparatus according to claim 11, wherein
the discrimination parameter is a threshold used for discriminating the body movement noise contained in the electroencephalography signal on a basis of an autocorrelation value of a common component in canonical correlation analysis between a singular mode function of the electroencephalography signal and a delayed singular mode function.

**15.** The information processing apparatus according to claim 1, further comprising

a body movement analysis unit that analyzes a body movement of the user, wherein

the noise reduction processing control unit controls the noise reduction processing on a basis of the identification result for the body movement noise and an analysis result for the body movement of the user.

**16.** The information processing apparatus according to claim 15, wherein
the noise reduction processing control unit stops the noise reduction processing in a case of determining that the user is in a rest state on a basis of the identification result for the body movement noise and the analysis result for the body movement of the user.

**17.** The information processing apparatus according to claim 15, wherein
the body movement noise identification unit identifies, on a basis of the electroencephalography signal and the analysis result for the body movement of the user, the body movement noise contained in the electroencephalography signal.

**18.** An information processing method, comprising:

identifying, on a basis of an electroencephalography signal of a user, body movement noise contained in the electroencephalography signal; and
controlling, on a basis of an identification result for the body movement noise, noise reduction processing of reducing the body movement noise contained in the electroencephalography signal.

**19.** A program that causes a computer to execute processing of:

identifying, on a basis of an electroencephalography signal of a user, body movement noise contained in the electroencephalography signal; and
controlling, on a basis of an identification result for the body movement noise, noise reduction processing of reducing the body movement noise contained in the electroencephalography signal.

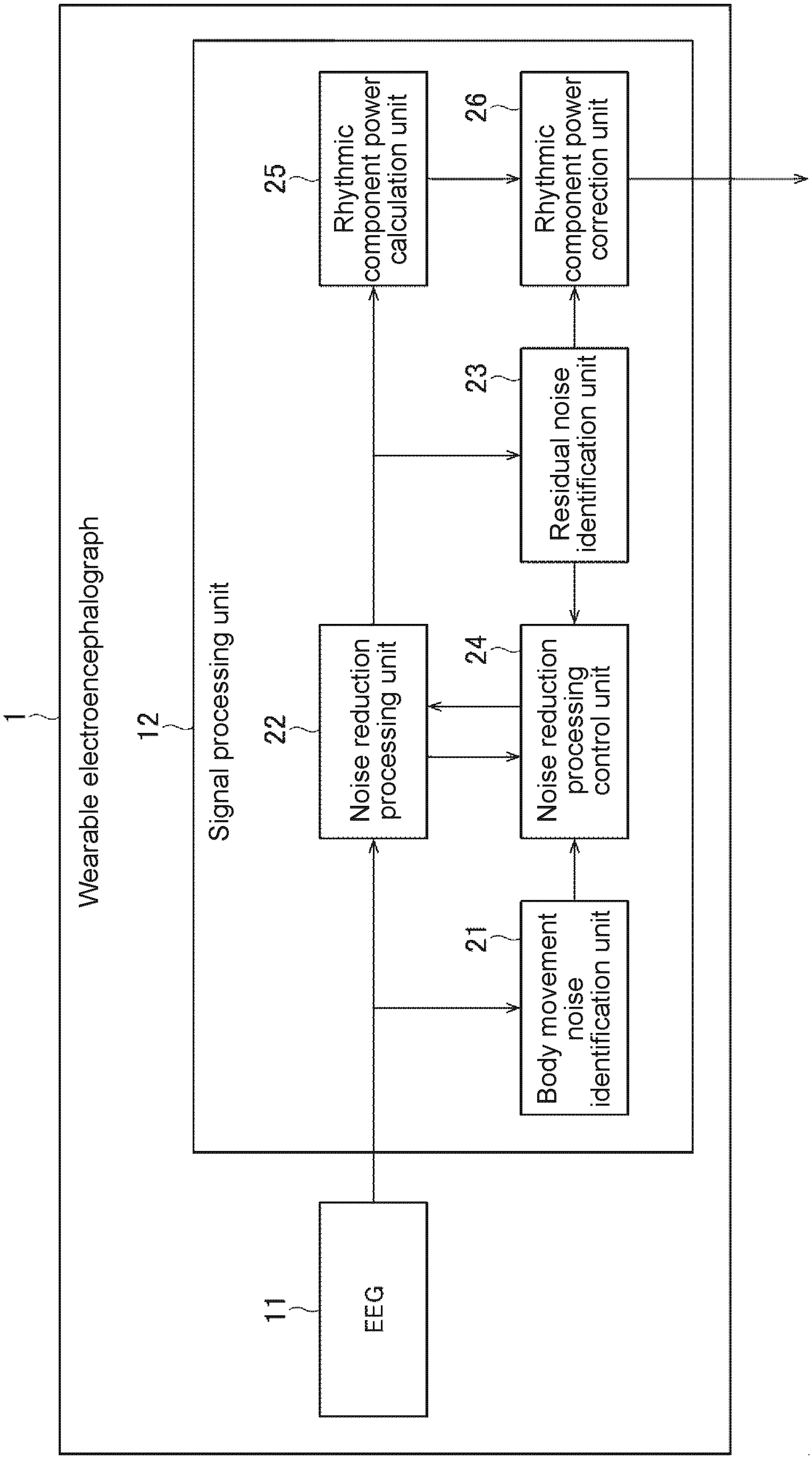
1
Wearable electroencephalograph
11
EEG
12
Signal processing unit
21
Body movement noise identification unit
22
Noise reduction processing unit
23
Residual noise identification unit
24
Noise reduction processing control unit
25
Rhythmic component power calculation unit
26
Rhythmic component power correction unit

FIG.1

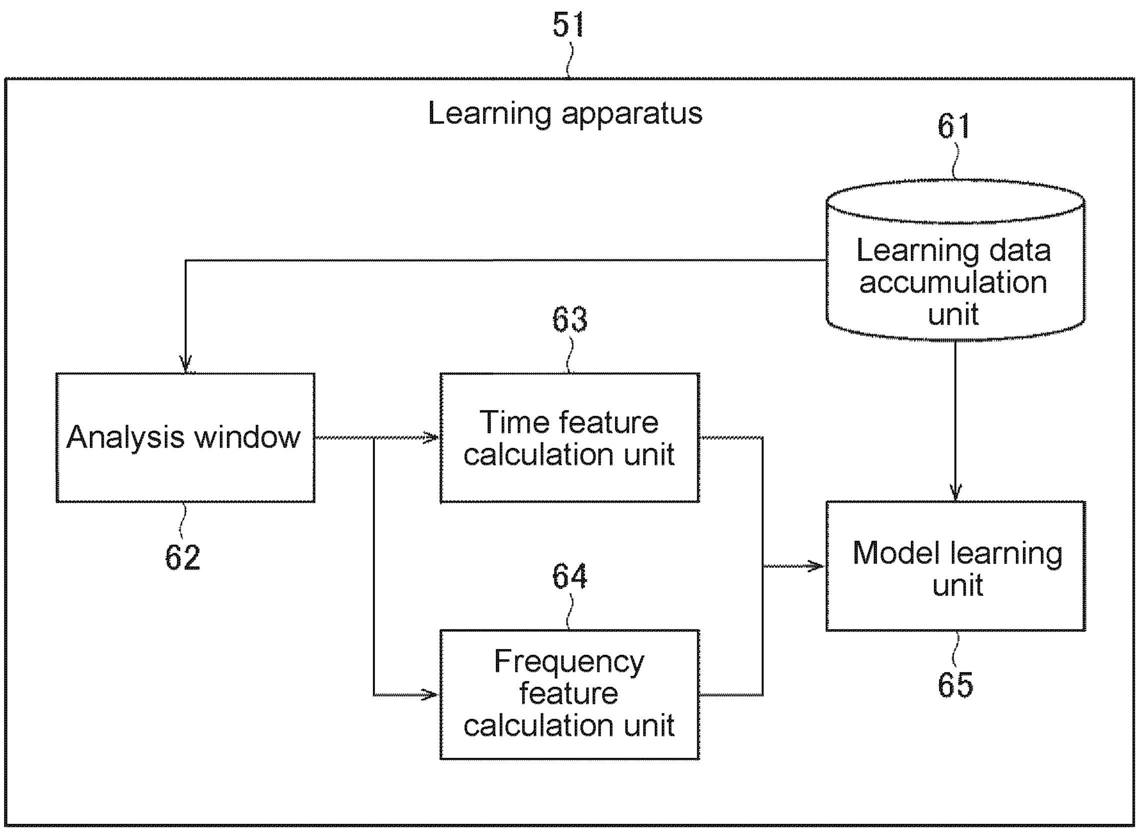
51
Learning apparatus
61
Learning data accumulation unit
63
Analysis window
Time feature calculation unit
62
64
Model learning unit
Frequency feature calculation unit
65

FIG.2

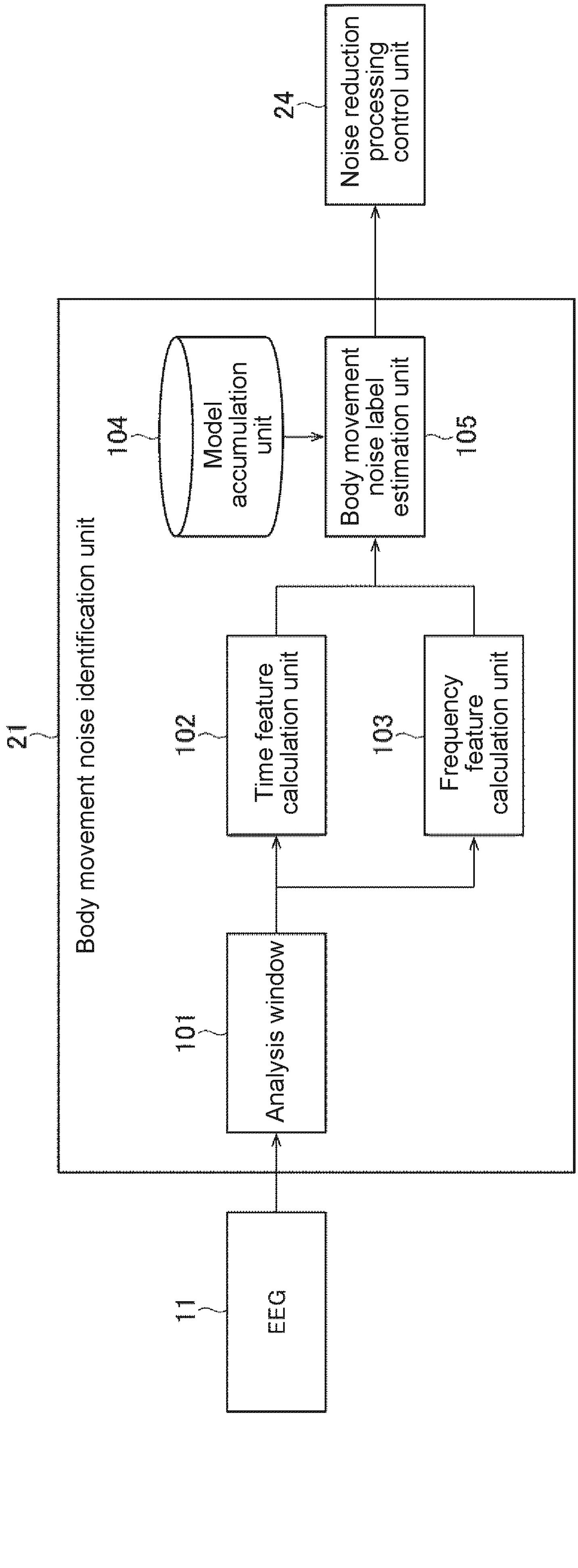
11
EEG
21
Body movement noise identification unit
101
Analysis window
102
Time feature calculation unit
103
Frequency feature calculation unit
104
Model accumulation unit
105
Body movement noise label estimation unit
24
Noise reduction processing control unit

FIG.3

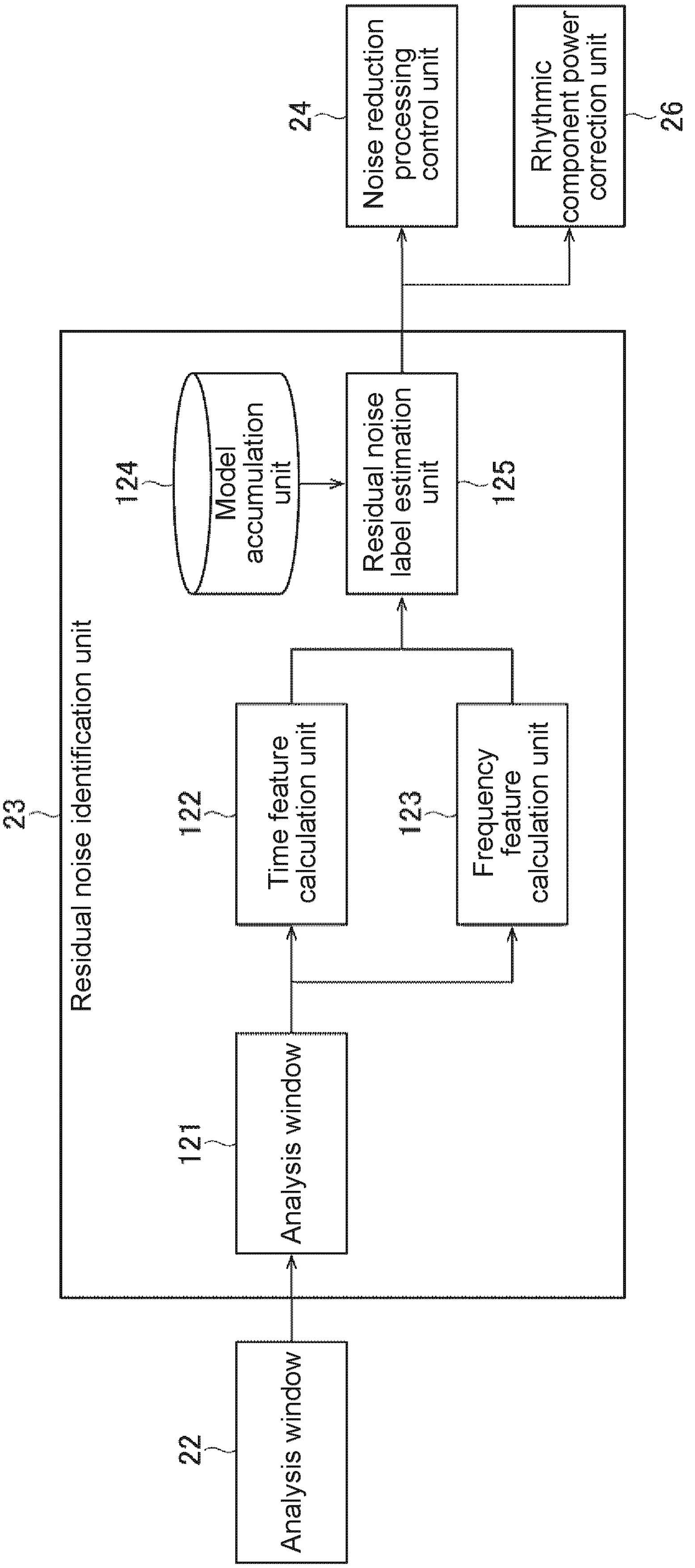
22
Analysis window
23
Residual noise identification unit
121
Analysis window
122
Time feature calculation unit
123
Frequency feature calculation unit
124
Model accumulation unit
125
Residual noise label estimation unit
24
Noise reduction processing control unit
26
Rhythmic component power correction unit

FIG.4

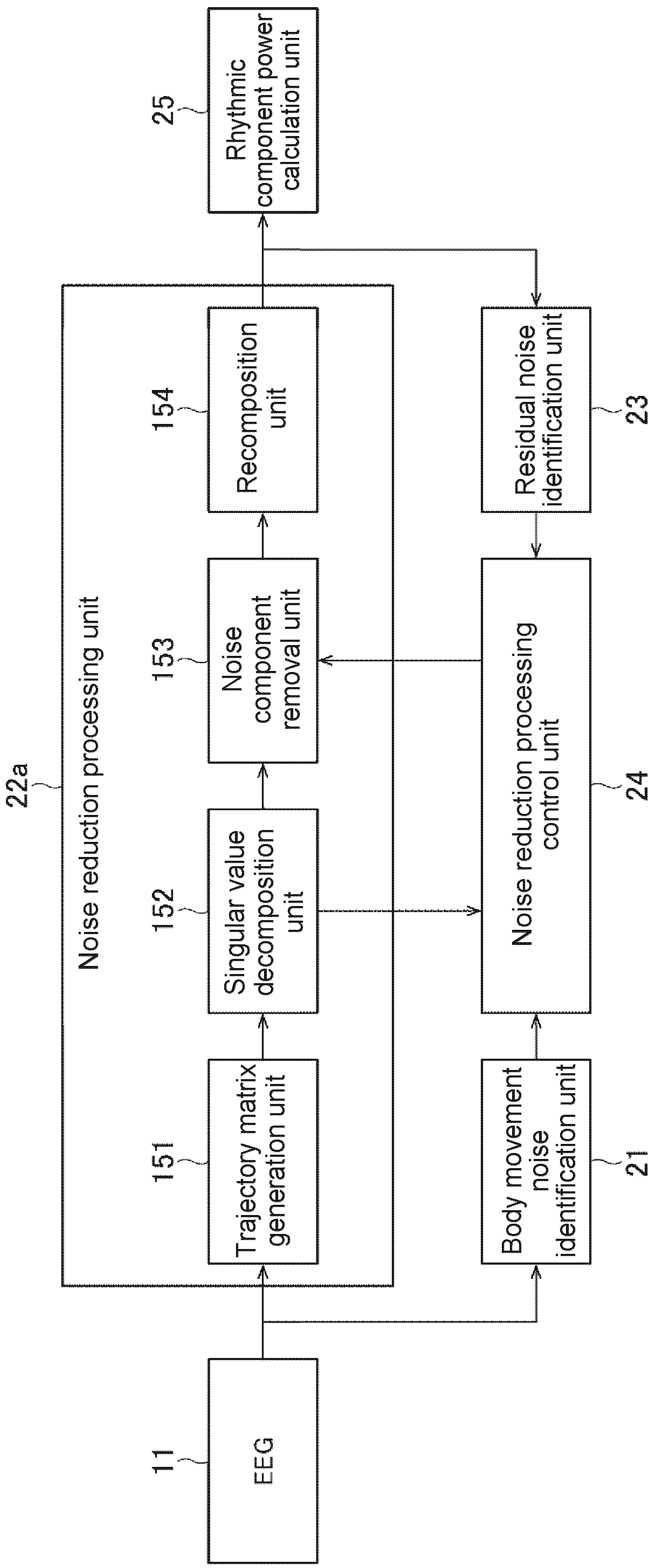
11
EEG
22a
Noise reduction processing unit
151
Trajectory matrix generation unit
152
Singular value decomposition unit
153
Noise component removal unit
154
Recomposition unit
25
Rhythmic component power calculation unit
21
Body movement noise identification unit
24
Noise reduction processing control unit
23
Residual noise identification unit

FIG.5

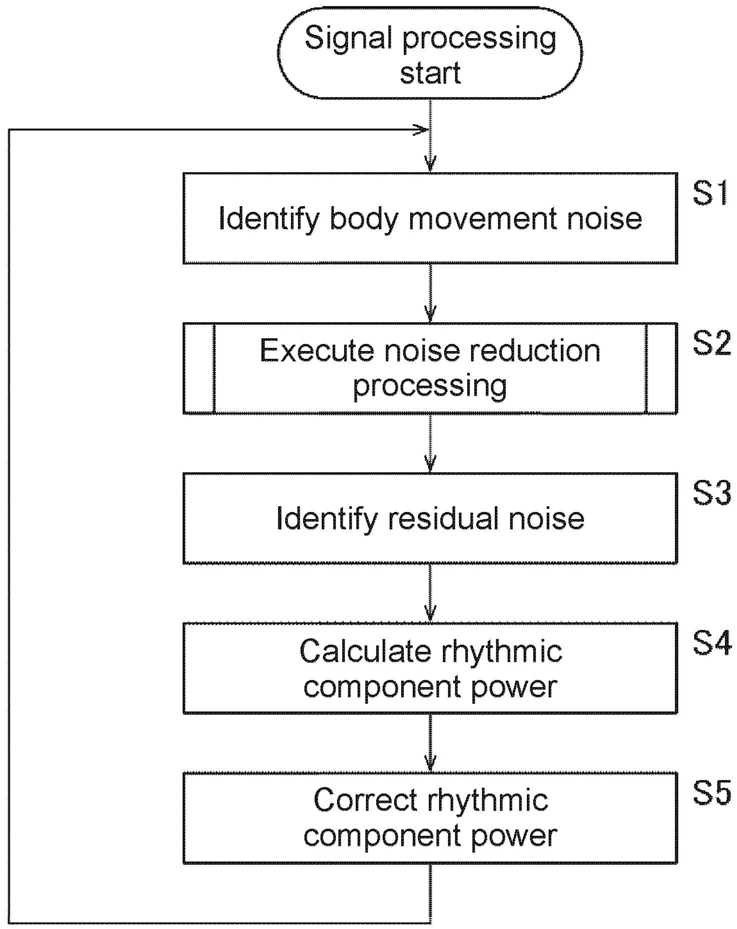
Signal processing start
Identify body movement noise
S1
Execute noise reduction processing
S2
Identify residual noise
S3
Calculate rhythmic component power
S4
Correct rhythmic component power
S5

FIG.6

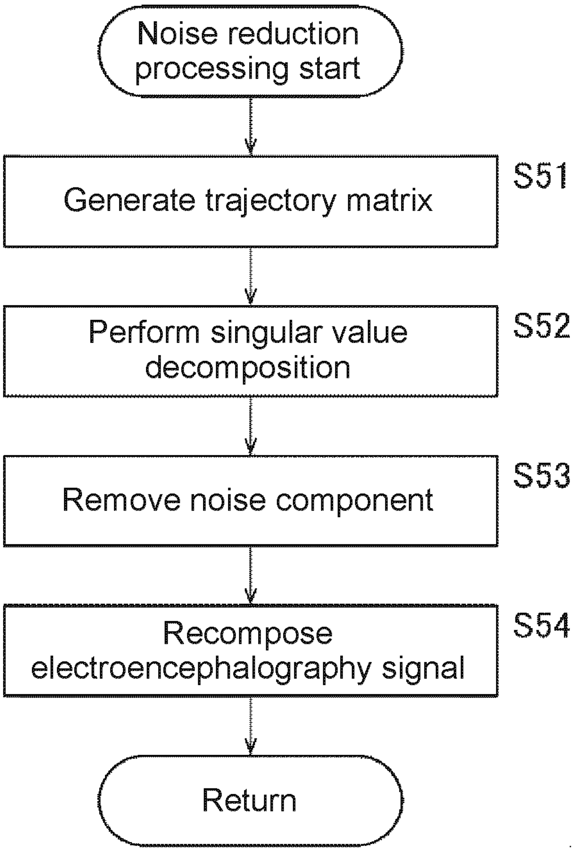
Noise reduction processing start
Generate trajectory matrix
S51
Perform singular value decomposition
S52
Remove noise component
S53
Recompose electroencephalography signal
S54
Return

FIG.7

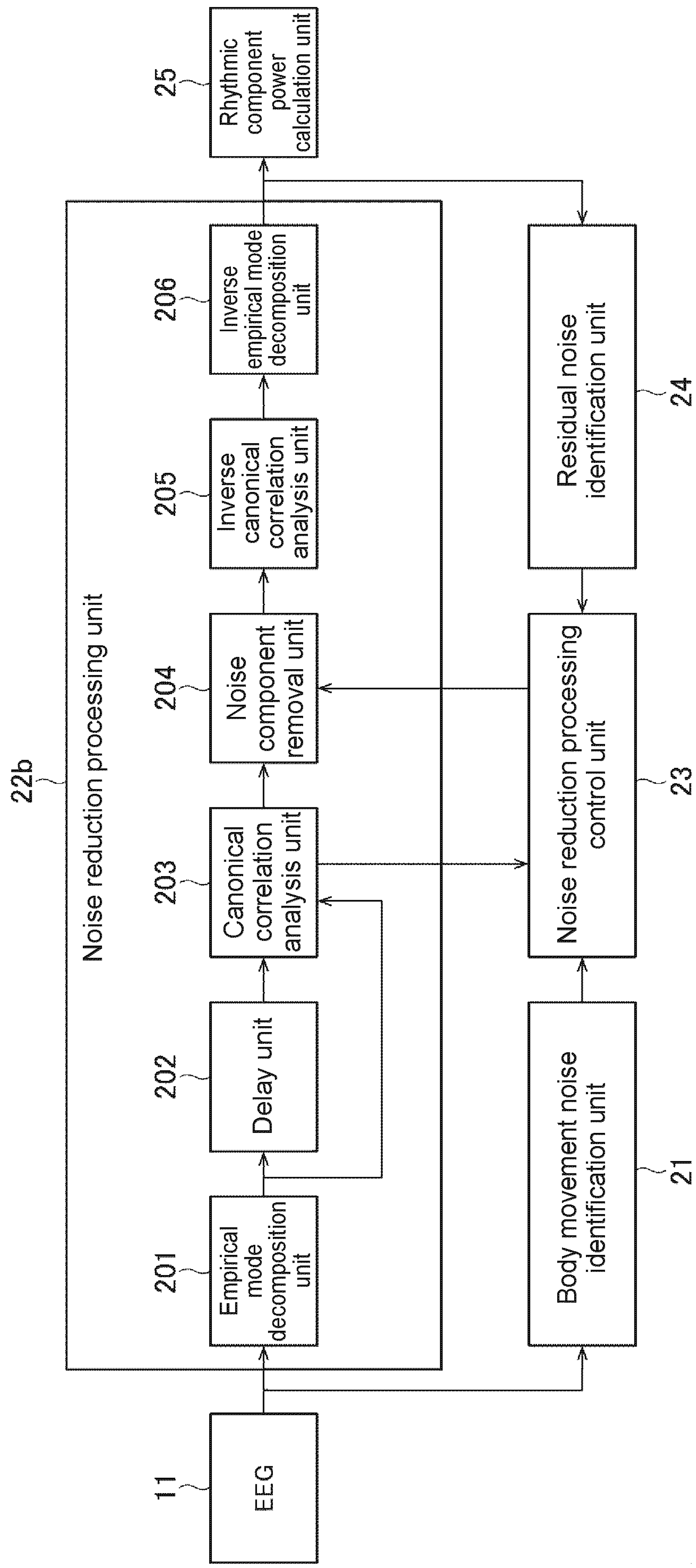
22b
Noise reduction processing unit
11
EEG
201
Empirical mode decomposition unit
202
Delay unit
203
Canonical correlation analysis unit
204
Noise component removal unit
205
Inverse canonical correlation analysis unit
206
Inverse empirical mode decomposition unit
25
Rhythmic component power calculation unit
21
Body movement noise identification unit
23
Noise reduction processing control unit
24
Residual noise identification unit

FIG.8

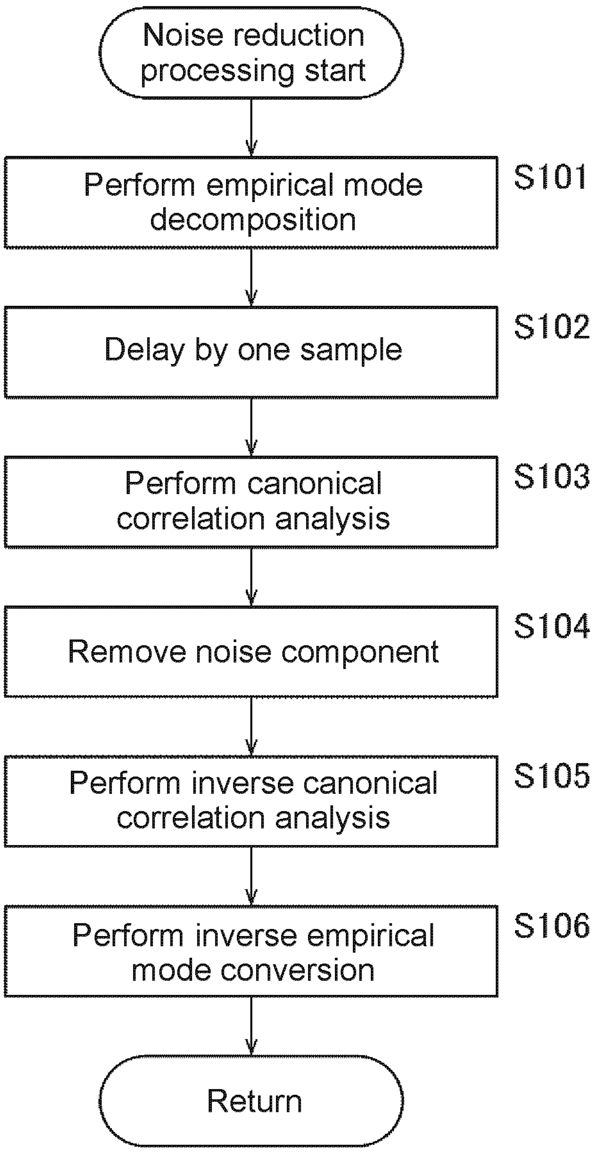
Noise reduction processing start
Perform empirical mode decomposition
S101
Delay by one sample
S102
Perform canonical correlation analysis
S103
Remove noise component
S104
Perform inverse canonical correlation analysis
S105
Perform inverse empirical mode conversion
S106
Return

FIG.9

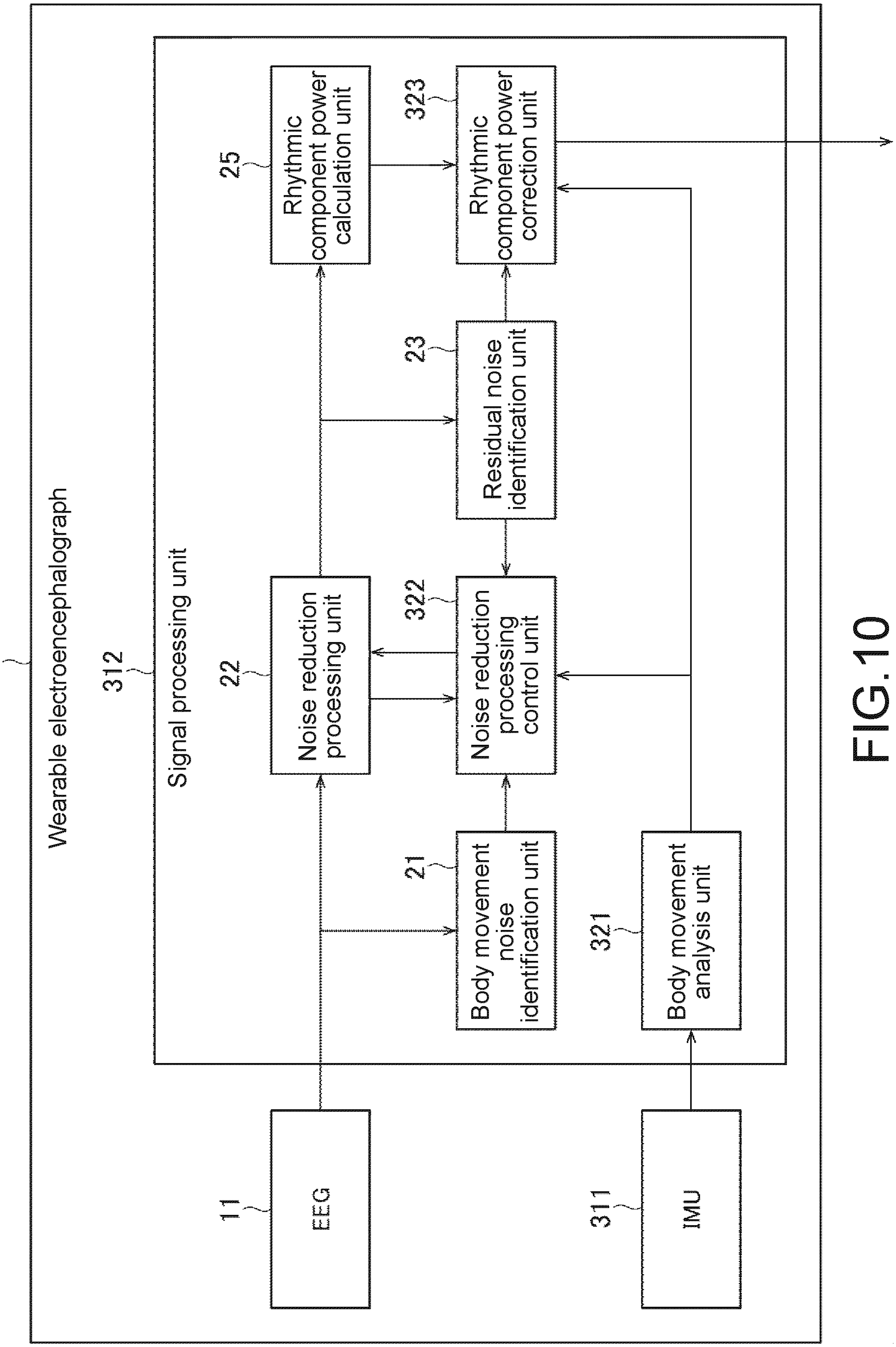
301
Wearable electroencephalograph
312
Signal processing unit
11
EEG
311
IMU
21
Body movement noise identification unit
321
Body movement analysis unit
22
Noise reduction processing unit
322
Noise reduction processing control unit
23
Residual noise identification unit
25
Rhythmic component power calculation unit
323
Rhythmic component power correction unit

FIG.10

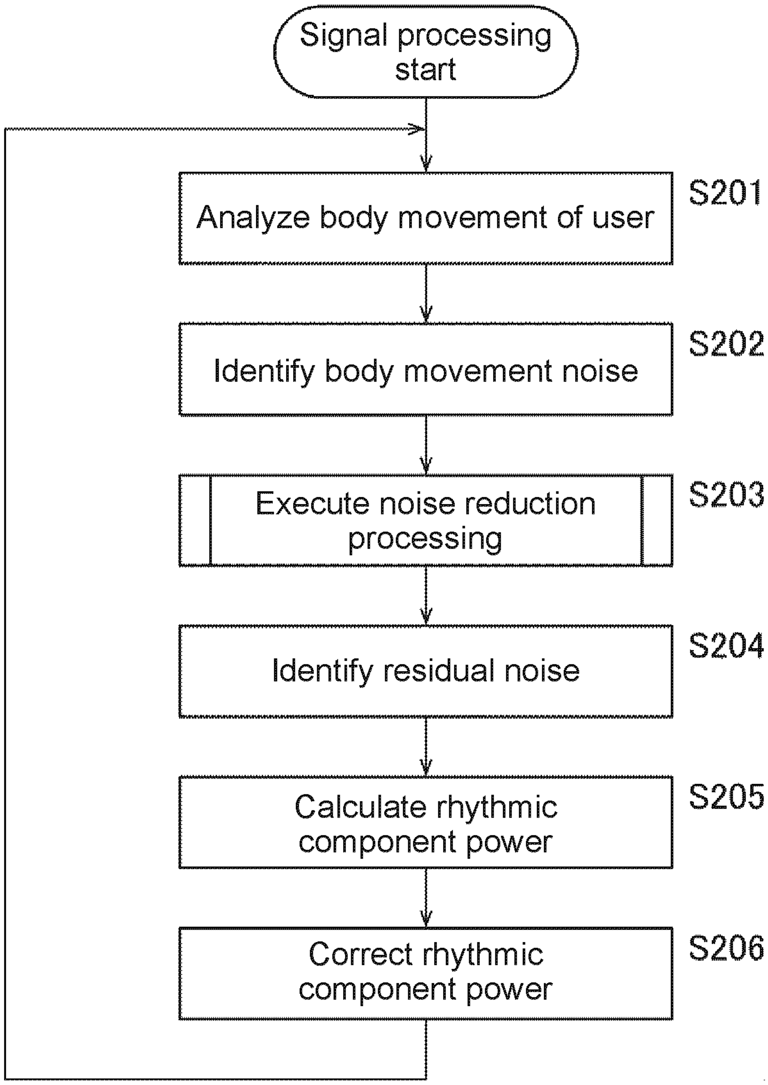
Signal processing start
S201
Analyze body movement of user
S202
Identify body movement noise
S203
Execute noise reduction processing
S204
Identify residual noise
S205
Calculate rhythmic component power
S206
Correct rhythmic component power

FIG.11

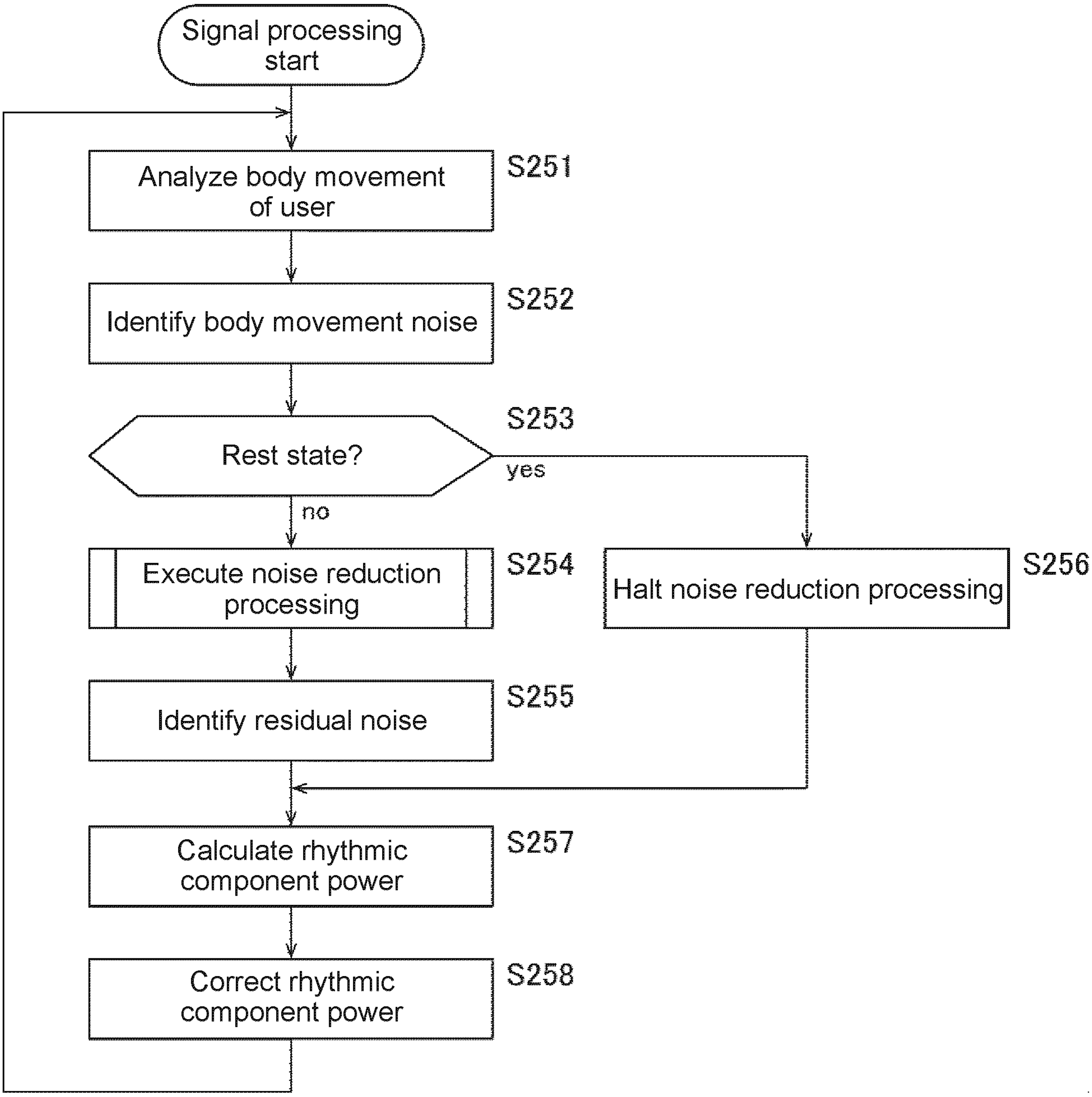
Signal processing start
Analyze body movement of user
S251
Identify body movement noise
S252
Rest state?
S253
yes
no
Execute noise reduction processing
S254
Halt noise reduction processing
S256
Identify residual noise
S255
Calculate rhythmic component power
S257
Correct rhythmic component power
S258

FIG.12

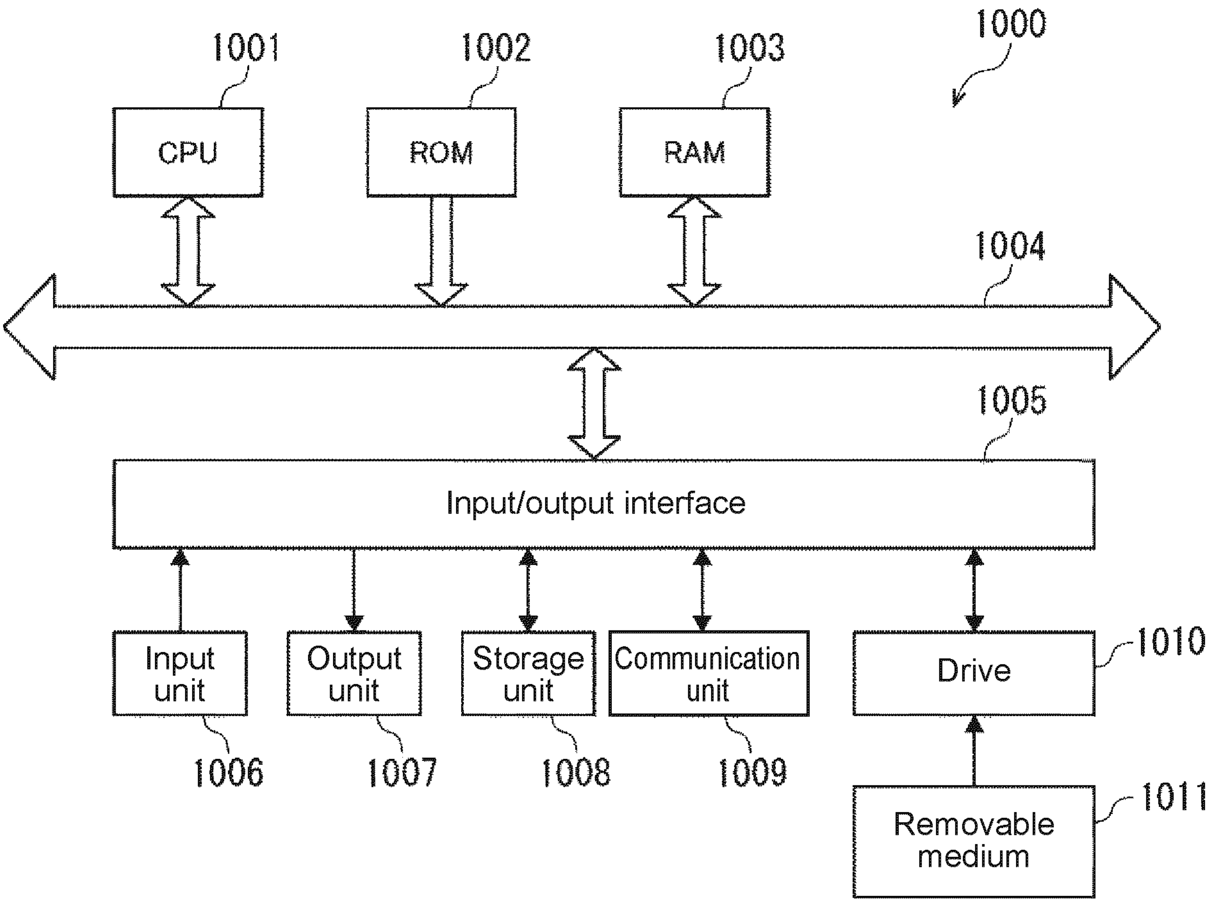
1000
1001
1002
1003
CPU
ROM
RAM
1004
1005
Input/output interface
Input unit
Output unit
Storage unit
Communication unit
Drive
1010
1006
1007
1008
1009
Removable medium
1011

FIG.13

## INTERNATIONAL SEARCH REPORT

International application No.
**PCT/JP2022/010204**

**A. CLASSIFICATION OF SUBJECT MATTER**

***A61B 5/374***(2021.01)i
FI: A61B5/374

According to International Patent Classification (IPC) or to both national classification and IPC

**B. FIELDS SEARCHED**

Minimum documentation searched (classification system followed by classification symbols)

A61B5/05-5/0538, 5/24-5/398

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2019-503746 A (KONINKLIJKE PHILIPS N.V) 14 February 2019 (2019-02-14)<br>paragraphs [0023]-[0026] | 1, 10-11, 13-14, 18-19<br>2-9, 12, 15-17 |
| Y | JP 2006-521888 A (MEDTRONIC INC) 28 September 2006 (2006-09-28)<br>paragraphs [0060]-[0062] | 2-9 |
| Y | JP 2017-42386 A (SEIKO EPSON CORP) 02 March 2017 (2017-03-02)<br>paragraphs [0062], [0086]-[0091] | 3-7, 15-17 |
| Y | WO 2017/135116 A1 (NIPPON TELEGRAPH AND TELEPHONE CORPORATION) 10 August 2017 (2017-08-10)<br>paragraphs [0017]-[0019] | 7 |
| Y | WO 2011/074186 A1 (PANASONIC CORPORATION) 23 June 2011 (2011-06-23)<br>paragraph [0130] | 12 |
| A | JP 2002-529118 A (CONSOLIDATED RESEARCH OF RICHMOND, INC) 10 September 2002 (2002-09-10)<br>paragraphs [0210]-[0214] | 1-19 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 March 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**

International application No.
**PCT/JP2022/010204**

**C. DOCUMENTS CONSIDERED TO BE RELEVANT**

| Category* | Citation of document, with indication, where appropriate, of the relevant passages | Relevant to claim No. |
|---|---|---|
| A | WO 2020/017162 A1 (SONY CORPORATION) 23 January 2020 (2020-01-23) paragraphs [0069]-[0075] | 1-19 |

Form PCT/ISA/210 (second sheet) (January 2015)

**INTERNATIONAL SEARCH REPORT**
**Information on patent family members**

International application No.
**PCT/JP2022/010204**

| Patent document cited in search report | Publication date (day/month/year) | Patent family member(s) | Publication date (day/month/year) |
|---|---|---|---|
| JP 2019-503746 A | 14 February 2019 | US 2018/0360376 A1<br>paragraphs [0030]-[0031]<br>WO 2017/108766 A1<br>EP 3393337 A1<br>CN 108471947 A | |
| JP 2006-521888 A | 28 September 2006 | US 2004/0193065 A1<br>paragraphs [0058]-[0060]<br>WO 2004/089205 A1<br>EP 1613209 A1 | |
| JP 2017-42386 A | 02 March 2017 | (Family: none) | |
| WO 2017/135116 A1 | 10 August 2017 | US 2019/0038164 A1<br>paragraphs [0034]-[0036]<br>CN 108601546 A | |
| WO 2011/074186 A1 | 23 June 2011 | US 2012/0029336 A1<br>paragraph [0158]<br>CN 102448371 A | |
| JP 2002-529118 A | 10 September 2002 | US 5813993 A<br>column 46, line 4 to column 47, line 14<br>WO 2000/018471 A1<br>EP 1124611 A1 | |
| WO 2020/017162 A1 | 23 January 2020 | JP 2020-10803 A<br>CN 112399823 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019503746 A